(19) Europäisches Patentamt
European Patent Office
Office européen des brevets

(11) EP 2 021 333 B1

(12) EUROPEAN PATENT SPECIFICATION

(45) Date of publication and mention of the grant of the patent:
**14.07.2010 Bulletin 2010/28**

(21) Application number: **07789580.3**

(22) Date of filing: **22.05.2007**

(51) Int Cl.:
***C07D 309/38*** (2006.01) ***C07D 215/22*** (2006.01)
***C07D 405/12*** (2006.01) ***C07D 407/12*** (2006.01)
***A61K 31/4704*** (2006.01) ***A61K 31/352*** (2006.01)

(86) International application number:
**PCT/IB2007/002179**

(87) International publication number:
**WO 2007/135565 (29.11.2007 Gazette 2007/48)**

(54) **ANTI-PROLIFERATIVE COMPOUNDS DERIVING FROM A 3-ARYL-COUMARINE OR 3-ARYL-QUINOLIN-2-ONE AND USES THEREOF**

PROLIFERATIONSHEMMENDE VERBINDUNGEN AUS EINEM 3-ARYL-COUMARIN ODER EINEM 3-ARYL-CHINOLIN-2-ON UND ANWENDUNGEN DAVON

COMPOSÉS ANTIPROLIFÉRATIFS DÉRIVÉS DE LA 3-ARYL-COUMARINE OU DE LA 3-ARYL-QUINOLIN-2-ONE ET LEUR UTILISATIONS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **22.05.2006 EP 06290821**

(43) Date of publication of application:
**11.02.2009 Bulletin 2009/07**

(73) Proprietor: **EOS - Ethical Oncology Science S.p.A.**
**20121 Milano (IT)**

(72) Inventors:
- **SCHAPIRA, Matthieu**
  **F-69001 Lyon (FR)**
- **LAMIGEON, Cyrile**
  **F-38080 L'ISLE D'ABEAU (FR)**
- **GUTMANN, Mathieu**
  **F-69004 Lyon (FR)**
- **BARTHELAIX, Audrey**
  **F-69009 Lyon (FR)**
- **HUGO, Nicolas**
  **F-69001 Lyon (FR)**
- **COLAS, Pierre**
  **F-69370 St Didier au Mont d'Or (FR)**

(74) Representative: **Minoja, Fabrizio**
**Bianchetti Bracco Minoja S.r.l.**
**Via Plinio 63**
**20129 Milano (IT)**

(56) References cited:
**WO-A-00/03990** **WO-A-2004/072043**
**WO-A2-00/39120** **WO-A2-03/020698**

- **KIRKIACHARIAN S ET AL: "Synthesis and binding affinity to human alpha and beta estrogen receptors of various 7-hydroxycoumarins substituted at 4- and 3,4- positions" IL FARMACO, ROME, IT, vol. 59, no. 12, December 2004 (2004-12), pages 981-986, XP004676521 ISSN: 0014-827X**

EP 2 021 333 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Cancer is one of the most widespread diseases around the world. Although the treatments proposed to patients are making progress, medicaments remain in many cases poorly effective, the treatments are usually difficult to bear due to dramatic side effects, and often do not avoid the chirurgical ablation of the tissue or organ affected by tumor cells. Patient death is still a frequent occurrence.

Indeed, despite recent advances in the diagnosis and treatment of cancer, tumor cell progression and metastasis still constitute a major cause of morbidity and mortality in cancer patients. Understanding the molecular and cellular mechanisms that contribute to tumor formation, progression and metastasis is a major challenge in cancer research, as is the need to inhibit the proliferation of tumoral cells.

Most of the anticancer drugs available to date kill tumor cells by activating an endogenous biochemical pathway for cell suicide, known as programmed cell death or apoptosis. Apoptosis is a genetically-programmed cell death. Apoptosis is also important in controlling cell number and proliferation as part of normal development Although some chemotherapeutic agents do not induce apoptosis directly, this programmed cell death is often the final common pathway for cells that have been damaged in various ways by cytotoxic drugs.

However, many malignant cells develop defects in the regulation of genes that control the apoptotic pathway, thus rendering such cells resistant to chemotherapy.

A number of mechanisms have been proposed for tumor insensitivity to the effects of conventional chemotherapy agents, including multidrug resistance gene expression, membrane pump extrusion of chemotherapeutic agents, tumor hypoxia, decreased tumor angiogenesis, and unfavorable cell cycle kinetics. Resistance to apoptosis is another mechanism via which tumors may both avoid programmed senescence and death as well as chemotherapy effects.

It has been reported that the development of apoptosis resistance can lead to an impaired response to conventional chemotherapeutic agents.

Although there are multiple pathways via which a cell can enter apoptosis, it appears that the majority of chemotherapy drugs induce apoptosis via effects on the mitochondrion (cytochrome c activation of cellular caspases) rather than cell surface death receptors such as FasL. The BCL-2 family of proteins are the major regulators of mitochondrial apoptotic homeostasis. The interplay between phosphorylating events, mediated largely by the phosphatidyl inositol 3 (P13) kinase system, as well as the balance achieved between pro-apoptotic and anti-apoptotic BCL-2 proteins determines whether or not the cell enters or avoids the process of programmed cell death.

The BCL-2 family of proto-oncogenes is a critical regulator of apoptosis, whose expression frequently becomes altered in human cancers, including lymphomas. Bcl-2 was the first member of this family to be identified, by virtue of its involvement in chromosomal translocation commonly found in B-cell non-Hodgkin's lymphoma (NHL).

The BCL-2 family is a large family of proteins subdivided into two subfamilies, which share some regions of homology known as BCL-2 Homology (BH) regions, a first class being anti-apoptotic, and a second class being pro-apoptotic. Their main mechanism of action is thought to be the regulation of mitochondrial membrane permeability. Pro-apoptotic members of the BCL-2 superfamily increase mitochondrial membrane permeability, whereas anti-apoptotic members of the family act to oppose this increase.

Pro-apoptotic members of the BCL-2 family include Bax, Bak, Bad, Bcl-Xs, Bid, Bik, Bim, and Hrk, and anti-apoptotic members include Bcl2, Bcl-xL, Bcl-W, Bfl-1, and Mcl-1.

Anti-apoptotic BCL-2 members act as repressors of apoptosis by blocking the release of cytochrome-c, whereas pro-apoptotic members act as promoters. There are several possible mechanisms by which members of the BCL-2 family could regulate mitochondrial membrane permeability.

The anti-apoptotic members of the BCL-2 family are overexpressed in many cancers even without the presence of chromosomal translocations. Increased expression of anti-apoptotic BCL-2 members causes resistance to chemotherapeutic drugs and radiation therapy, while decreasing anti-apoptotic BCL-2 members expression may promote apoptotic responses to anticancer drugs. In addition, overexpression of Bcl-2, an anti-apoptotic member, may result in accumulation of cells in the Go phase of cell cycle division and contribute to chemoresistance.

With regard to Bcl-xL, an anti-apoptotic member of the BCL-2 family, it has been previously demonstrated that:

- Bcl-xL is a major anti-apoptotic protein
- Bcl-xL (and other BCL-2-like proteins) is found over-expressed in many cancers
- Basal expression of Bcl-xL correlates strongly with chemoresistance to various classes of anti-tumor molecules.

Therefore, small molecule inhibitors of anti-apoptotic activity of Bcl-xL are expected to exert an anti-tumor effect on a wide range of cancers, either alone or in combination with existing cytotoxic agents.

The BCL-2 family of proteins, and especially Bcl-xL are thus attractive targets for drug design. As pivotal regulators of apoptotic cell death, the logic of manipulating BCL-2 functions for anti-tumor effects is one of the molecular targets proposed for cancer therapeutics. Moreover, elevated levels of anti-apoptotic proteins have been demonstrated

in virtually every type of human cancer.

Initial efforts have been centered on disrupting protein-protein interactions within the BCL-2 homology (BH) family. Substantial progress in this task has been made using molecular modeling and drug leads.

Different molecules targeting proteins of the BCL-2 family are presently in preclinical or clinical trials, *inter alia*:

- ABT-737 (Oltersdorf et al, 2005): Molecular targets: Bcl-xL, Bcl-2, Bcl-w. ABT-737 induces apoptosis of small cell lung carcinoma (SCLC) and lymphoid cells and induces SCLC tumor regression in mouse models.
- IPI-983L (http:/www.ipi.com/science bio.html) :Molecular targets : Bcl-xL, Bcl-2.
- Gossypol (Mohammad RM et al, 1995) :Molecular targets : BCL-2 like proteins. This molecule has been shown to inhibit growth of breast and prostate tumor cell lines *in vitro.*
- GX15-070 (Contractor R. et al, and Galan PP et al): Molecular targets: BCL-2-like proteins (Kd for Bcl-w and MCL-1 - 0.5mM). This molecule has been shown to inhibit growth of melanoma, breast, cervical, prostate, colon tumor cells. It has however a low MTD (maximal tolerated dose of approx 16mg/kg).

WO03/020698 A2 describes a 2-quinolinone derivative substituted in the 3-position by a 2-methoxy-4-nitrophenyl group, namely 3-(2-methoxy-4-nitrophenyl)-4-hydroxy-7-methoxy-1H-quinolin-2-one. The compound is endowed with antiproliferative activity.

3-arylquinolin-2-ones having antiproliferative activity against clonogenic tumor cells are described in WO00/03990 A.

WO2004/072043 A describes androgen receptor antagonists based on the quinolin-2-one or chromen-2-one (coumarin) structure which are useful to treat or alleviate conditions associated with inappropriate expression of the androgen receptor, such as prostate cancer.

The present inventors have now obtained a novel family of potent anti-cancer molecules targeting Bcl-xL. The family of molecules is derived from a 3-aryl-coumarine or 3-aryl-quinolin-2-one. The present Invention Is directed to these compounds and to their uses in therapy, especially for treating any type of cancer.

In the context of the present invention, the following terms are defined according to the following definitions:

**Apoptosis** is defined as programmed cell death, and Involves the systematic disassembly of a cell. In contrast to necrosis, which is a form of cell death that results from acute cellular injury,
apoptosis is carried out in an ordered process that generally confers advantages during an organism's life cycle.

The main function of apoptosis Is to dispose of a cell without causing damage or stress to neighbouring cells.

Apoptosis involves the systematic dissassembly of the cell into apoptotic bodies, which are taken up by phagocytes. This avoids the inflammatory response associated with necrosis. Apoptosis can be detected using the assay described in example 7 of the experimental part. **Anti-apoptotic:** Something that prevents, decreases or delays apoptosis.

**Pro-apoptotic:** Something that activates or accelerates apoptosis.

**Proliferation:** multiplication or increase in number. In biology, cell proliferation occurs by cell division.

**Anti-proliferative:** something that prevents, decreases or delays proliferation of cells; therefore after a treatment with an anti-proliferative compound, the number of cells remains static, or increases less rapidly than without treatment, or decreases. An anti-proliferative activity may be due to a cytostatic effect, to a cytotoxic effect, or to a combination of both effects.

The anti-proliferative activity of a compound can be tested and measured according to the protocol detailed in the experimental part of the description (see example 2C). A molecule is said to have anti-proliferative activity according to the present invention if the number of cells is significantly diminished (from a statistical point of view) in a sample treated during 72 hours with said molecule with respect to the number of cells in a sample treated during 72 hours with a control, or untreated.

**Cytotoxic:** something producing a lethal effect on cells. A compound is considered as cytotoxic, if after treatment for 72 hours with said compound the number of cells is significantly lower (from a statistical point of view) than the number of cells initially seeded. Example 2A of the specification details a potential protocol for determining the cytotoxic activity of a compound. According to the invention, a compound having cytotoxic properties is an example of an anti-proliferative compound.

Cytotoxicity may lead to the death of the cell *inter alia* by necrosis or apoptosis.

**Cytostatic:** something inhibiting or suppressing cellular division. According to the invention, a compound having cytostatic properties is an example of an anti-proliferative compound. **Cancer:** Any of various malignant neoplasms characterized by the proliferation of anaplastic cells that tend to invade surrounding tissue and metastasize to new body sites.

**Anticancer** or **anticancerous:** Effective in treating cancer. **Tumor:** An abnormal growth of tissue resulting

from uncontrolled, progressive multiplication of cells and serving no physiological function; a neoplasm.

**Antitumor** or **antitumoral:** Counteracting or preventing the formation of malignant tumors; anticancer.

**Alkyl** represents straight and branched carbon chains that contain from one to twelve carbon atoms, preferably one to six carbon atoms; most preferably 1 to 5. A lower alkyl contains one to four carbon atoms, it is thus a $C_1$, $C_2$, $C_3$ or $C_4$ carbon chain.

**Cycloalkyl** represents saturated carbocyclic rings branched or unbranched of from 3 to 20 carbon atoms, preferably 3 to 7 carbon atoms; most preferably 5 or 6; said carbocyclic ring being optionally substituted with one or more of halo, alkyl, hydroxy, alkoxy, phenoxy, $CF_3$, amino, alkylamino, dialkylamino, -COOR, $-NO_2$ (wherein R is H or alkyl), C≡N, OH, $O-CH_3$, $CO-NH_2$, CO-OH, $CO-OCH_3$ or $O-CO-CH_3$; said carbocyclic ring being preferably mono- or di-substituted. A cycloalkyl possesses preferably less than 200 atoms or less than 100 atoms, even preferably less than 60 atoms, or less than 40 atoms.

**Heterocycloalkyl** represents a saturated, branched or unbranched carbocylic ring containing from 3 to 15 carbon atoms, preferably from 4 to 6 carbon atoms, which carbocyclic ring is interrupted by 1 to 3 hetero groups selected from -O-, -S- or - NR- (wherein R can be, for example, $-C(O)N(H)_2$, $-CH_2C(O)N(H)_2$, -CHO, -C(O)-O-H, alkyl, aryl, aralkyl, cycloalkyl, heterocycloalkyl or heteroaryl). Preferably, a heterocycloalkyl is a carbocyclic ring interrupted by 1 or 2 hetero groups. A heterocycloalkyl possesses preferably less than 200 atoms or less than 100 atoms, even preferably less than 60 atoms, or less than 40 atoms.

Preferred heterocycloalkyls are furan, thiophene, pyrrole, thiazole, pyrrazole and oxazole. **Aryl** (including the aryl portion of aryloxy and aralkyl) represents a carbocyclic group containing from 6 to 15 carbon atoms and having at least one aromatic ring, said carbocyclic group being optionally substituted with one or more of halo, alkyl, heterocycloalkyl (e.g. N-alkyl-piperazine, morpholine,...), hydroxy, alkoxy, phenoxy, $CF_3$, amino, alkylamino, dialkylamino, -COOR (wherein R is H, alkyl, aryl, heteroaryl or cycloalkyl), $NO_2$, C≡N, OH, $O-CH_3$, $CO-NH_2$, CO-OH, $CO-OCH_3$ or $O-CO-CH_3$; said carbocylcic group is preferably mono- or di-substituted. An aryl possesses preferably less than 200 atoms or less than 100 atoms, even preferably less than 60 atoms, or less than 40 atoms.

**Heteroaryl** represents a cyclic group, optionally substituted, having at least one heteroatom selected from O, S or N, said heteroatom interrupting a carbocyclic ring structure and having a sufficient number of delocalized pi electrons to provide aromatic character, with the aromatic heterocyclic groups preferably containing from 2 to 14 carbon atoms. The aromatic heterocyclic group is optionally substituted with one or more of the substituents mentioned in the definition of aryl. Said carbocyclic ring is preferably interrupted by 1 or 2 heteroatoms. A heteroaryl possesses preferably less than 200 atoms or less than 100 atoms, even preferably less than 60 atoms, or less than 40 atoms.

**Aralkyl** (shortened form of **arylalkyl)** represents groups made up of both aryl and alkyl groups.

**Legend of figures:**

**FIG.1:** (1A and 1B) Inhibition of cell proliferation *in vitro.*
This graph represents the percentage of growth of fourteen different populations of cells, in presence of increasing concentration of a compound of the invention: compound (01).
**FIG.2:** Inhibition of tumor growth *in vivo.*
This graph represents the growth of tumor size (by reference to the initial size of the tumor) grafted to mice receiving different treatments during 15 days.
Rhombus: evolution of the growth of the tumor measured in mice receiving the vehicle (without active ingredient) intra-peritoneally (2QD)
Triangle: evolution of the growth of the tumor measured in mice receiving the compound (01) intra-peritoneally at a dosage of 18 mg/kg (2QD).
Circle: evolution of the growth of the tumor measured in mice receiving taxol intra-peritoneally at 8 mg/kg (Q3D x5; d1; d4; d7; d10; d13).
Cross: evolution of the growth of the tumor measured in mice receiving a combination of compound (01) at a dosage of 18 mg/kg 20D and taxol at a dosage of 8 mg/kg Q3D x5 intra-peritoneally.
**FIG.3:** Inhibition of the interaction between Bcl-xL and BF8. a peptide aptamer that targets the hydrophobic groove of BCL-2 family members.

This picture is a Western blot analysis showing the presence, or absence of the protein fusion 6xHis-Bcl-xL, captured by GST-BF8, in presence or in absence of a peptide of the invention, specifically compounds (01) and (02).
C-: control peptide aptamer (does not interact with Bcl-xL).
C+: 6xHis-Bcl-xL captured by GST-BF8, in presence of 5% DMSO.
(02): 6xHis-Bcl-xL captured by GST-BFB, in presence of 50$\mu$M of compound 02 (in 5% DMSO).

(01): 6xHis-Bcl-xL captured by GST-BF8. in presence of 50μM of compound 01 (in 5% DMSO).

FIG.4; This figure depicts the time course of cellular effects induced by 0.5μM of compound 01 in U937 cells.

X-axis: time of exposure to compound (01), in hours.

Y-axis: % of positive cells

By using virtual screening *in silico*, the present inventors have identified a molecule capable of interacting *in silico,* with the Bcl-xL protein, whose three-dimensional structure is known (Sattler et al). This molecule has then been tested *in vitro* and *in vivo* and has prove to be an efficient anti-proliferative agent.

Specifically, the compound designated 01 inhibits the proliferation of a large panel of human cancer cells *in vitro.* Compound 01 also inhibits the growth of a non-small cell lung carcinoma in an *in vivo* xenograft model in the mouse.

Lead optimization has allowed the identification of a family of molecules, structurally related to compound 01, having comparable properties, useful in the treatment of cancer.

The compound 01 is (2*E*)-3-(2-furyl)-*N*-({[3-methoxy-4-(2-oxo-2*H*-chromen-3-yl)poenyl]amino}carbonothioyl) acrylamide.

A number of derivatives, keeping the core structure, have now been tested by the inventors, in order to enhance the advantageous anti-proliferative properties of the compound 01 and improve its suitability as a drug. All these molecules essentially possess the following structural core:

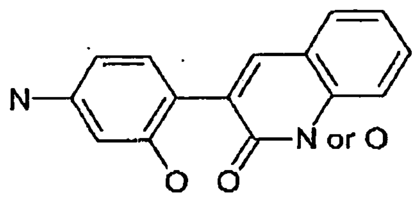

The present invention is thus directed to compounds having one of the general formula :

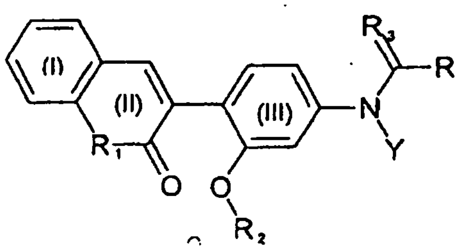

formula (a)

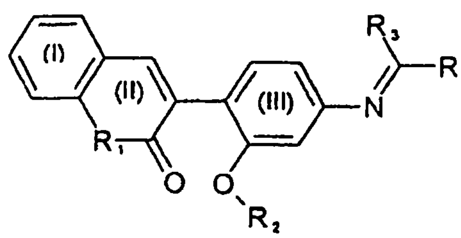

formula (a')

or

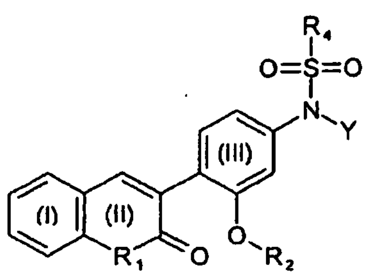

formula (b)

- wherein the cycles (I), (II) and (III) may be optionally substituted on 1, 2, 3 or 4 of the available carbons with a group chosen amongst: Br, Cl, F, I, OH, O-$CH_3$, COOH, and $(CH_2)_m$-$R_9$, wherein $R_9$ represents H, $CH_3$, OH, O-$CH_3$, COOH, $NH_3$, CO-$NH_2$, NH-CO-phenyl, an alkyl, an heteroaryl or an heterocycloalkyl and m=1, 2, 3 or 4.
- wherein Y represents H, $CH_3$ or CO-$CH_3$ ;
- wherein $R_1$ represents O, $NR_{18}$ with $R_{16}$ being H or an alkyl having from 1 to 4 carbon atoms, or N-$(CH_2)_p$-$R_7$, with $R_7$ representing OH, $COOR_{16}$, CO-$NHR_{16}$, $NHR_{16}$, O-$CH_2R_{16}$, CN, O-CO-$CH_2R_{16}$, CO-heterocycloalkyl, a cycloalkyl, a heterocycloalkyl, an aryl or a heteroaryl, possibly substituted with one or more of the followings radicals:

an alkyl, an aryl, a heteroaryl, a cycloalkyl, a heterocycloalkyl, F, Cl, $NH_2$, C≡N, OH, CO-$NH_2$, O-$CH_3$, CO-OH, CO-$OCH_3$ or O-CO-$CH_3$, and p=0, 1, 2, 3, 4, 5 or 6;

- wherein $R_2$ represents H or an alkyl with less than 4 carbon atoms,
- wherein $R_3$ represents $S_1$ O or NH in formula (a), or S-$(CH_2)_q$-$R_{10}$ or NH-$(CH_2)_q$-$R_{10}$, in formula (a'), wherein $R_{10}$ has the same meaning as $R_9$ and wherein q= 0, 1, 2, 3 or 4;
- wherein $R_4$ represents an alkyl, aryl, arylalkyl, heteroaryl, heteroarylalkyl, cycloalkyl, heterocycloalkyl, or CH=CH-$R_{11}$. wherein $R_{11}$ has the same meaning as $R_4$, and the molecular weight of $R_4$ is less than 500 Da,
- and wherein,

if $R_3$ represents O, then R is N'-$R_5$, or O-$R_5$, wherein $R_5$ has the same meaning as $R_4$ and N* represents NH or N($CH_3$);

if $R_3$ represents NH, S-$(CH_2)_q$-$R_{10}$ or NH-$(CH_2)_q$-$R_{10}$, then R is $R_6$, or N*-CO-$R_6$, wherein $R_6$ has the same meaning as $R_4$ and N* represents NH or N($CH_3$); and

if $R_3$ represents S, then

- Ris N*-$(CH_2)_n$-$R_{12}$, wherein $R_{12}$ has the same meaning as $R_7$ , n=0, 1, 2, 3 or 4 and N* represents NH or N ($CH_3$), or
- R is N*-CO-$R_8$, with -$R_8$ representing a linear or branched alkyl, with the exception of tert-butyl, or -$R_8$ represents -$(CH_2)_8$-$C_4H_3O$, with s=1, 2, 3 or 4, or $R_8$ represents a phenyl radical mono-substituted in the para position, with an alkyl, -F, -Cl, -$NH_2$, -C≡N, -OH, -CO-$NH_2$, -O-$CH_3$, -CO-OH, -CO-$OCH_3$ or -O-CO-$CH_3$ and N* represents NH or N($CH_3$), or
- R is N*-CO-$R_{17}$, with -$R_{17}$ representing an alkyl, aryl, arylalkyl, heteroaryl, heteroarylalkyl, cycloalkyl, heterocycloalkyl, or CH=CH-$R_{18}$. wherein $R_{18}$ has the same meaning as $R_{17}$, and the molecular weight of $R_{17}$ is less than 500 Da, N* represents NH or N($CH_3$) and $R_1$ is not O,

as well as base and acid addition salts of said compounds, the geometric isomers, enantiomers and diastereoisomers thereof.

Each substitutent is chosen independently of the others.

According to an embodiment of the invention, the molecule has the general formule (a). According to another embodiment, a molecule of the invention has the general formula (a'). According to still another embodiment, a molecule of the invention has the formula (b).

The overall molecular mass of the compound is preferably less than 1000 Da. Preferably, the molecular weight is less than 800 Da, preferably less than 700, 650 or 600 Da. Indeed, the compound is preferably capable of passing (passively) the cell membrane. Therefore, the molecular weight of the compound is if possible minimized.

Preferably, a compound according to the invention has anti-protiferative properties, it may thus be useful as an antitumoral agent The compounds of the invention have preferably an anti-proliferative effect mainly targeted on tumour cells, cancerous cells or immortalized cells, i.e. the anfi-proliferative (or cytotoxic effect) is at least 10% greater on a population of tumour or cancerous cells than on a population of normal cells (not immortalized). For example, after incubating a population of cells with a compound according to the invention, the decrease in the proliferation rate of the cells is 10% greater on a population of tumour or cancerous cells than on a population of normal cells. The difference in the effect on a population of tumour or cancerous cells, with a population of normal cells is preferably at least 20%, at least 30% or at least 50% or even more.

Anti-proliferdtive activity can be tested and measured according Lu the protocol detailed in the experimental part of the description (see example 2C).

More preferably, said compounds have cytotoxic activity or pro-apoptotic activity. Preferably, this activity is preferentially targeted on tumour calls, Similarly, this means that the cytotoxic or pro-apoptotic effect is at least 10% greater on a population of tumour or cancerous cells than on a population of normal cells.

Pro-apoptotic activity can be tested and measured according to the protocol detailed in the experimental part of the description (example 7).

Preferably, a compound as defined according to the invention is for use in therapy, in a prophylactic or curative treatment.

The compounds as defined are indeed useful for eliminating cancerous cells; they are thus very useful in the treatment of cancers, leukaemia or any type of tumours.

The compounds according to the invention are preferably to be administered to a patient, which is an animal, preferably a mammal and most preferably a human being. Therefore, substituents likely to render the compound immunogenic to an animal, or likely to give rise to secondary toxic effects, or likely to decrease the maximal tolerated dose

are preferably avoided. Moreover, the substituents are preferably chosen in order to improve solubility of the compound, especially the solubility in body fluids of an animal.

For determining preferred substituents in the formula (a), (a') and (b), the criteria to be applied are the following, classified by decreased significance:

- to confer an enhanced anti-proliferative activity to the whole compound; or
- to confer an anti-protiferative activity more precisely targeted on tumour cells to the whole compound; or
- to confer to the compound a less immunogenic or toxic character, or
- to confer a better solubility to the whole compound or
- to confer a metabolic protection to the compound thus increasing the bioavailability or the half-life, or
- having a smaller molecular weight

Different structures are preferred in the context of the present invention, namely $R_1$ is preferably an oxygen atom, in any of the formula (a), (a') or (b), or NH.

Independently of the nature of $R_1$ moiety, the $R_2$ moiety is preferably a methyl in any of the formula (a), (a') or (b). Preferably, $R_1$ is O and $R_2$ is $CH_3$ or $R_1$ is NH and $R_2$ is $CH_3$.

Compounds according to the invention have advantageously at least one of cycles (I) and (II) which is substituted. Suitable substituents particularly preferred in the context of this invention are substituents capable of increasing the antiproliferative activity of the compound, substituents capable of increasing the solubility of the compound, substituents capable of decreasing the antigenic action of the compound and very importantly substituents conferring a metabolic protection to the compound. Such potential substituents are known from the skilled man.

Preferably, cycle (I) alone, cycle (II) alone, or cycles (I) and (II) are substituted. Cycle (III) may also be substituted. Preferably, each of the cycles (I), (II) and (III) possesses only one additional substitution, in addition to the substitution already present in formula (a), (a') and (b).

Preferred substituents for cycles (I) and (II) are fluorine (F) and $(CH_2)_m$-$R_9$, with m=1 or 2 and {$R_9$) is

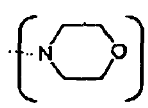 or 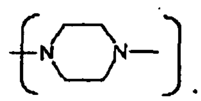.

Such a substituent may also be adopted for cycle (III).

According to another preferred embodiment of the invention, Y represents a hydrogen atom in formula (a) or (b).

According to other preferred embodiments of the invention, $R_1$ is O and/or -$R_2$ is -$CH_3$, in any of the formula (a), (a') and (b).

According to another embodiment, $R_1$ is N-$(CH_2)_p$-CO-N-methylpiperazine, preferably N-CO-N-methylpiperazine.

According to still another preferred embodiment $R_{16}$ is H or $CH_3$, and most preferably $R_{16}$ is H. According to still another embodiment, $R_{17}$ is a furan, a thiophene, a pyrrole, a thiazole, a pyrrazole or an oxazole moiety, $R_1$ being in this case not O; preferably $R_{17}$ is a furan moiety.

Preferred compounds of the invention correspond to compounds wherein in formula (a) $R_1$ is O, -$R_2$ is -$CH_3$, $R_3$ is O, Y is H, R is NH-$R_5$ and $R_5$ is an aryl, a cycloalkyl, an heterocylcoalkyl, an heteroaryl or an arylakyl, preferably $R_5$ is a phenyl or $R_5$ is a benzyl or -$R_5$ is -$C_6H_4$-$N(CH_3)_2$.

Other preferred compounds of the invention are those wherein in formula (a) $R_1$ is O, -$R_2$ is -$CH_3$, $R_3$ is NH, Y is H and
(R) is

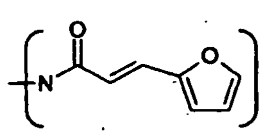 or 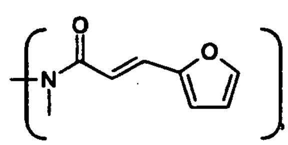

the furan moiety being optionally substituted with an alkyl having from 1 to 4 carbon atoms, $NH_3$, O, OH, CO-$NH_2$, O-$CH_3$, CO-OH, CO-$OCH_3$ or O-CO-$CH_3$. Alternatively, the furan moiety may be replaced by another heterocycloalkyl, for instance a thiophene, a pyrrole, a thiazole, a pyrrazole or an oxazole moiety, optionally substituted as mentioned above.

Optionally, $R_1$ may be NH instead of O.

Still other preferred compounds according to this first aspect of the invention are compounds, wherein in formula

(b): $R_1$ is O, $-R_2$ is $-CH_3$, Y is H and ($R_4$) is

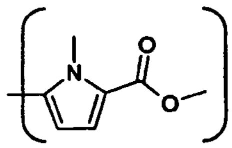 or 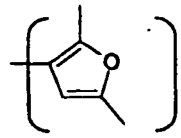 .

Other compounds preferred according to the present invention are compounds wherein in formula (a): $R_1$ is O or NH, $-R_2$ is $-CH_3$, $R_3$ is NH, Y is H and -R is $-NH-CO-C_6H_5$.

Other molecules which are preferred in the context of the present invention have the general formula (a) with the following features: $R_1$ is O, $R_2$ is H or $CH_3$, $R_3$ is S, Y is H, R is $NH-(CH_2)_nR_{12}$, n=0 and ($R_{12}$) is

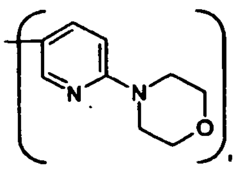, 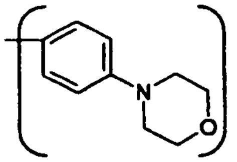, 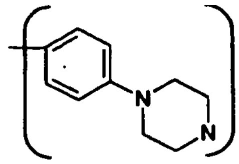, 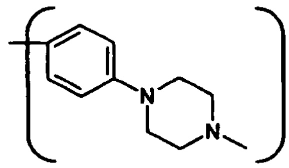,

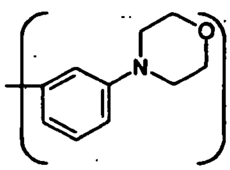, 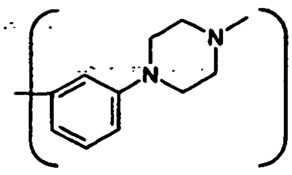, 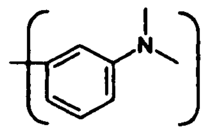 or 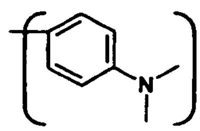.

Optionally, in the preceding formula, $R_3$ may be O instead of S.

Still other molecules also having a structure corresponding to the general formula (a) and which are particularly preferred in the context of this invention have the following features: $R_1$ is O, $-R_2$ is $-CH_3$, $R_3$ is S, Y is H, R is $NH-(CH_2)_n-R_{12}$, n=0 and $R_{12}$ is $C_6H_5$. Optionally, $R_3$ may be O instead of S.

Another class of preferred compounds correspond to formula (a) wherein $R_1$ is O, $-R_2$ is $-CH_3$, $R_3$ is S, Y is H, R is $NH-CO-R_8$ and $-R_8$ is $-CH_2CH_2C_4H_3O$ or ($R_8$) is

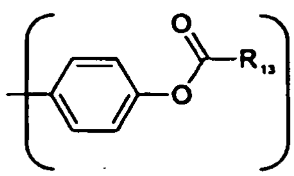

wherein $R_{13}$ is an alkyl in $C_1$ to $C_6$.

Alternatively, other preferred compounds of the invention are compounds, wherein in formula (a): . $R_1$ is O, $-R_2$ is $-CH_3$, $R_3$ is S, R is $NH-(CH_2)_n-R_7$, n=1 and $R_7$ is $-C_6H_5$.

For compounds of the invention having the general formula (a'), preferred substituents are the following: $R_1$ is O, $-R_2$ is $-CH_3$, $R_3$ is $S-CH_3$ and (R) is

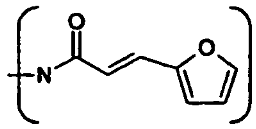 or 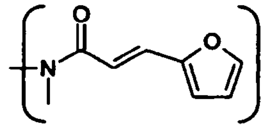 ,

the furan moiety being optionally substituted with an alkyl having from 1 to 4 carbon atoms, $NH_3$, O, OH, $CO-NH_2$. $O-CH_3$, CO-OH, $CO-OCH_3$ or $O-CO-CH_3$.

Alternatively, the furan moiety may be replaced by another heterocycloalkyl, especially a thiophene, a pyrrole, a thiazole, a pyrrazole or an oxazole moiety, optionally substituted as mentioned above.

Examples of particularly preferred compounds are:

Compound 03: 1-[3-methoxy-4-(2-oxo-2*H*-chromen-3-yl)phenyl]-3-phenylurea,
Compound 04: 1-[3-methoxy-4-(2-oxo-2*H*-chromen-3-yl)phenyl]-3-phenylthiourea,
Compound 05: 4-fluoro-*N*-({[3-methoxy-4-(2-oxo-2*H*-chromen-3-yl)phenyl]amino}carbonothioyl)benzamide,
Compound 06: 1-[4-(dimethylamino)phenyl]-3-[3-methoxy-4-(2-oxo-2 *H*-chromen-3-yl)phenyllurea,
Compound 07: *N*-(imino{[3-methoxy-4-(2-oxo-2*H*-chromen-3-yl)phenyl]amino}methyl)benzamide,
Compound 08: 1-[3-methoxy-4-(2-oxo-2*H*-chromen-3-yl)phenyl]-3-(6-morpholin-4-ylpyridin-3-yl)thiourea,
Compound 09: methyl 5-({[3-methoxy-4-(2-oxo-2*H*-chromen-3-yl)phenyl]amino}sulfonyl)-1-methyl-1 *H*-pyrrole-2-carboxylate,
Compound 10: methyl *N*-[(2*E*)-3-(2-furyl)prop-2-enoyl]-*N*'-[3-methoxy-4-(2-oxo-2*H*-chromen-3-yl)phenyl]-*N*-methylimidothiocarbamate,
Compound 12: (2*E*)-3-(2-furyl)-*N*-(imino{[3-methoxy-4-(2-oxo-2*H*-chromen-3-yl)phenyl]amino}methyl)acrylamide,
Compound 13: *N*-[3-methoxy-4-(2-oxo-2*H*-chromen-3-yl)phenyl]-2,5-dimethylfuran-3-sulfonamide,
Compound 14: methyl *N*-[(2*E*)-3-(2-furyl)prop-2-enoyl]-*N*'-[3-methoxy-4-(2-oxo-2*H*-chromen-3-yl)phenyl]imidothiocarbamate,
Compound 15: (2*E*)-3-(2-furyl)-*N*-({[3-methoxy-4-(2-oxo-1,2-dihydroquinolin-3-yl)phenyl]amino}carbonothioyl)acrylamide,
Compound 16: methyl *N*-(4-fluorobenzoyl)-*N*'-[3-methoxy-4-(2-oxo-2*H*-chromen-3-yl)phenyl]-*N*-methylimidothiocarbamate,
Compound 17: (2*E*)-3-(2-furyl)-*N*-({[3-methoxy-4-(1-methyl-2-oxo-1,2-dihydroquinolin-3-yl)phenyl]amino}carbonothioyl)acrylamide,
Compound 18: 1-[3-(dimethylamino)phenyl]-3-[3-methoxy-4-(2-oxo-2 *H*-chromen-3-yl)pheny]urea,
Compound 19: N'-((E)-3-furan-2-yl-acryloyl)-N-[3-methoxy-4-(2-oxo-2H-chromen-3-yl)-phenyl]-N-methyl-guanidine,
Compound 20: 1-[3-methoxy-4-(2-oxo-2H-chromen-3-yl)-Phenyl]-3-(4-morpholin-4-yl-phenyl)-thiourea,
Compound 21: 1-((E)-3-furan-2-yl-acryloyl)-3-{4-[1-(2-hydrcxy-ethyl)-2-oxo-1,2-dihydro-quinolin-3-yl]-3-methoxy-phenyl}-thiourea,
Compound 22: N-Benzoyl-N'-[3-methoxy-4-(2-oxo-1,2-dihydro-quinolin-3-yl)-phenyl]-guanidine,
Compound 23: 2,5-dimethyl-furan-3-sulfonic acid acetyl-[3-methoxy-4-(2-oxo-2H-chromen-3-yl)-phenyl]-amlde,
Compound 24: 1-((E)-3-furan-2-yl-acryloyl)-3-[3-methoxy-4-(2-oxo-2H-chromen-3-yl)-phenyl]-1,2-dimethyl-isothiourea, and
Compound 25: 1-(4-fluoro-benzoyl)-3-[3-methoxy-4-(2-oxo-2H-chromen-3-yl)-phenyl]-1,2-dimethyl-isothiourea.

The present invention is also directed to salts of the compounds of the invention, e.g. chlorhydrate salts, especially pharmaceutically acceptable salts of the compounds of the invention.

Moreover, the invention also concerns metabolites of the compounds of the invention, especially metabolites formed *in vivo.* Furthermore, the invention also relates to prodrugs of the compounds of the invention, which are transformed *in vivo* into said compounds.

Importantly, it is to be noted that "a compound of the invention" encompasses any stereoisomeric and/or tautomeric form thereof, as also mixtures of these different forms.

As mentioned above, a compound of the invention has preferably cytotoxic or cytostatic properties on tumor cells. Preferred tumoral cells are lung, prostate, breast, colon, melanoma, lymphoma and/ or myeloid tumor cells.

As mentioned above, a compound of the invention is preferably soluble in water and in different body fluids. With respect to the solubility in water, a compound of the invention can preferably be dissolved in water at a molarity above $50.10^{-6}$ $mol.L^{-1}$., preferably at molarity above $500.10^{-6}$ $mol.L^{-1}$ or even more preferably above $800.10^{-6}$ $mol.L^{-1}$ or around 1 $mmol.L^{-1}$ (i.e. around $10^{-3}$ $mol\text{-}L^{-1}$).

According to a preferred embodiment of the present invention, a compound as defined above is capable of

interacting, directly or indirectly with the anti-apoptotic protein Bcl-xL. It is referred that a compound of the invention binds the protein Bcl-xL, at least *in vitro,* preferably with a Kd less than 1μM. preferably less than 100nM, or even less, for example less than 20nM or less than 10nM. Such compounds are particularly preferred.

A suitable protocol for defining the Kd of the interaction between a molecule and the protein Bcl-xL is given in the experimental section of this application (see example 5).

The present invention also concerns a pharmaceutical formulation comprising a compound of the invention, such that the bioavailability of the compound is at least 20%, preferably at least 50%, even preferably at least 80%, 90% or 95%.

The present invention also concerns a composition comprising a compound as defined according to the invention, in association with at least one other active ingredient. Said other active ingredient may be a therapeutic agent of any type, for example an anti-inflammatory agent or a compound counterbalancing any side-effect of the compound of the invention (reducing, abolishing or delaying said potential side-effects).

Advantageously, the compound of the invention is used or provided in association with at least one other anti-tumor agent or chemotherapeutic or with radiation. It has indeed been observed by the inventors that the anti-cancer effect of a compound of the invention is enhanced when combined with another anti-cancer treatment. A composition may advantageously comprise an association of a compound of the invention with at least two different other chemotherapeutics, or at least three or more.

Suitable anti-tumor agents which can be combined with a compound according to the invention are for example: taxol (paclitaxel), etoposide, doxorubicin, cis-platin, retinoic acid, tamoxifen, vinblastin, vincristin, cyclophosphamide, topotecan, irinotecan, gemcitabine or rapamycin. This list is however not exhaustive, any other chemotherapeutic may be combined with a compound of the invention. Preferably, such a composition comprises a compound of the invention and another chemotherapeutic having also antl-proliferative property, i.e. having cytostatic and/or cytotoxic activity on tumour cells.

The invention also relates to the use of a composition as mentioned above for the manufacture of a medicament to be administered to a subject for treating cancer or any other pathology implying the abnormal growth of tissue resulting from uncontrolled, progressive multiplication of cells or the unwanted or pathological sustained proliferation of cells. The subject may be an animal, especially a mammal, and is preferably a human patient

The invention is also directed to a product containing a compound according to the invention and a further active agent, preferably an anti-tumor agent, as a combined preparation for simultaneous, separate or sequential use in therapy. The compound of the invention and the other anti-tumor agent are preferably the active ingredients of the product

A combined preparation for simultaneous therapy is defined as a treatment comprising administration of a compound of the invention and a further agent, preferably an anti-tumor agent, at the same time. Said compound and agent can be packaged together or as two separate entities to be given simultaneously.

A combined preparation for sequential therapy is defined as a treatment comprising the successive administration of a compound of the invention alone followed by the administration of a further agent alone, preferably an anti-tumor agent, or in the reverse order.

A combined preparation for separate therapy is defined as a treatment comprising administration of a compound of the invention and administration of a further agent, preferably an anti-tumor agent, wherein both therapies are concomitant, the administrations being however not all made simultaneously.

The present invention also concerns a pharmaceutical composition comprising a compound according to the invention and a pharmaceutically acceptable carrier. Contrary to an active ingredient, a pharmaceutically acceptable carrier is an excipient, devoid of therapeutic activity by itself. The skilled man in the pharmaceutical domain is able to define potential pharmaceutically acceptable carriers to be combined with a compound of the invention, depending on the chosen mode of administration. Different carriers may be envisaged and incorporated together in the pharmaceutical composition. Moreover, a compound of the invention can be combined with more than one acceptable carrier, for example with at least two or three, or more. Other ingredients may be added in a pharmaceutical composition of the invention, e.g. flavours, dyes, which are active or inactive from a therapeutic point of view.

As mentioned above, preferred additional ingredients are other anticancer agents and especially anti-cancer agents having anti-proliferative activity, for example at least one other compound of the invention.

According to a preferred embodiment of the invention, the pharmaceutical composition contains a compound of the invention, a suitable carrier and taxol. Respective proportions of each ingredient are to be determined by the skilled man in the domain.

A pharmaceutical composition of the Invention can be designed for an administration via intralesional, intraperitoneal, intramuscular or intravenous injection; via infusion; liposome-mediated delivery; topical, nasal, oral, anal, subcutaneous, vaginal, sublingual, uretheral, transdermal; intrathecal, ocular or otic delivery.

The mode of administration is to be determined depending on the pathology to be treated. Indeed, a different mode of administration is to be envisaged for treating for example a solid tumour and metastases.

The present invention is also directed to a compound of the invention for the manufacture of a medicament to be administered to a subject for treating cancer or other pathologies characterized by the abnormal growth of tissue resulting from uncontrolled, progressive multiplication of cells or by unwanted or pathological sustained proliferation of cells.

The subject to be treated is preferably a mammal, especially cats, dogs, horses and humans. Particularly preferred patients are humans.

According to this embodiment of the invention, the medicament to be prepared is to be administered via intralesional, intraperitoneal, intramuscular or intravenous injection; via infusion; liposome-mediated delivery; topical, nasal, oral, anal, subcutaneous, vaginal, sublingual, uretheral, transdermal, intrathecal, ocular or otic delivery. However, an alternative mode of administration may also be envisaged.

The invention also relates to the use of a compound of the invention for the manufacture of a medicament to be administered simultaneously, separately or sequentially with at least one other active agent, preferably an anti-tumor agent or a chemotherapeutic, to a subject for treating cancer or other pathologies characterized by the abnormal proliferation of cells. As mentioned above, the invention is not limited to the addition of only one other anti-tumor agents and several different agents can enter in the composition of a medicament as defined. Examples of potential other anti-tumor agents are: taxol (paclitaxel), etoposide, doxorubicin, cis-platin, retinoic acid, tamoxifen, vinblastin, vincristin, cyclophosphamide, topotecan, irinotecan, gemcitabine or rapamycin. This list is however not exhaustive.

The invention also concerns a process for inhibiting cell proliferation comprising contacting a cell with a compound of the invention. Such a process can be Carried out *in vivo* or *ex vivo*, but is preferably carried out *in vitro.*

## EXAMPLES

**Example 1:** Synthesis of compounds of the invention.

**1.A** Synthesis of courmarine derivatives

The main steps and conditions to be applied are summarized in the following reaction scheme. $^1$R and $^2$R are potential substituents of cycles (I) and (III) in formula (a), (a'), (b) and (c) as defined above. $^3$R, $^6$R and $^7$R represent potential substituents in order to obtain compounds according to formula (a), (a'), (b) or (c) as defined above.

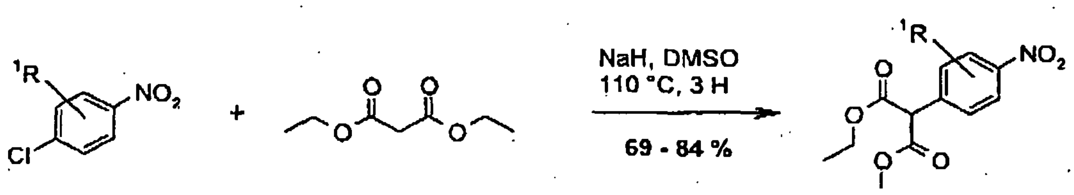


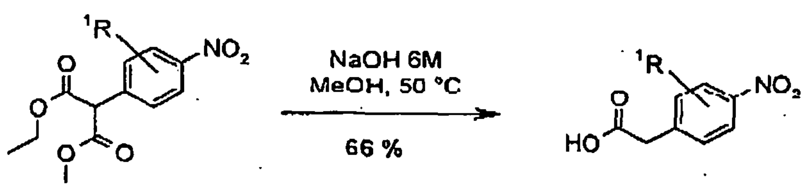


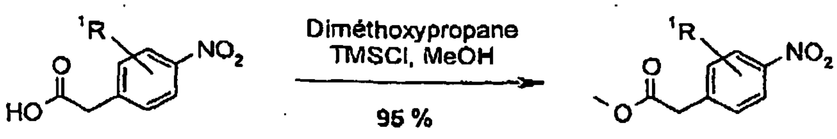

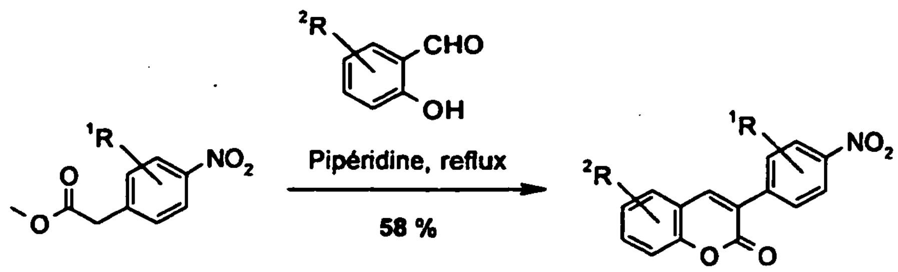


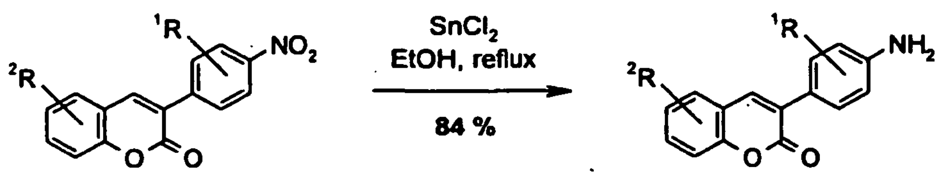


or

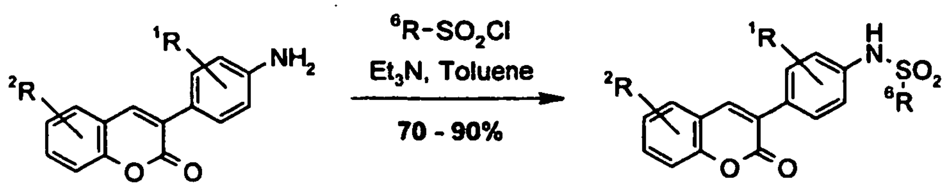


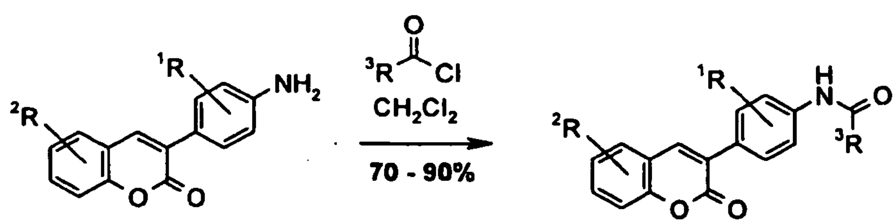


or

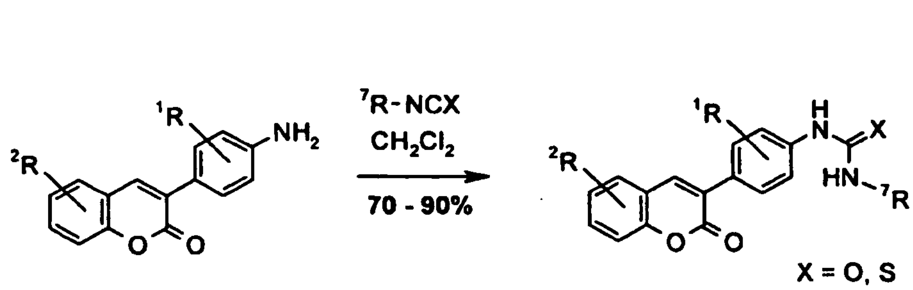


More specifically:

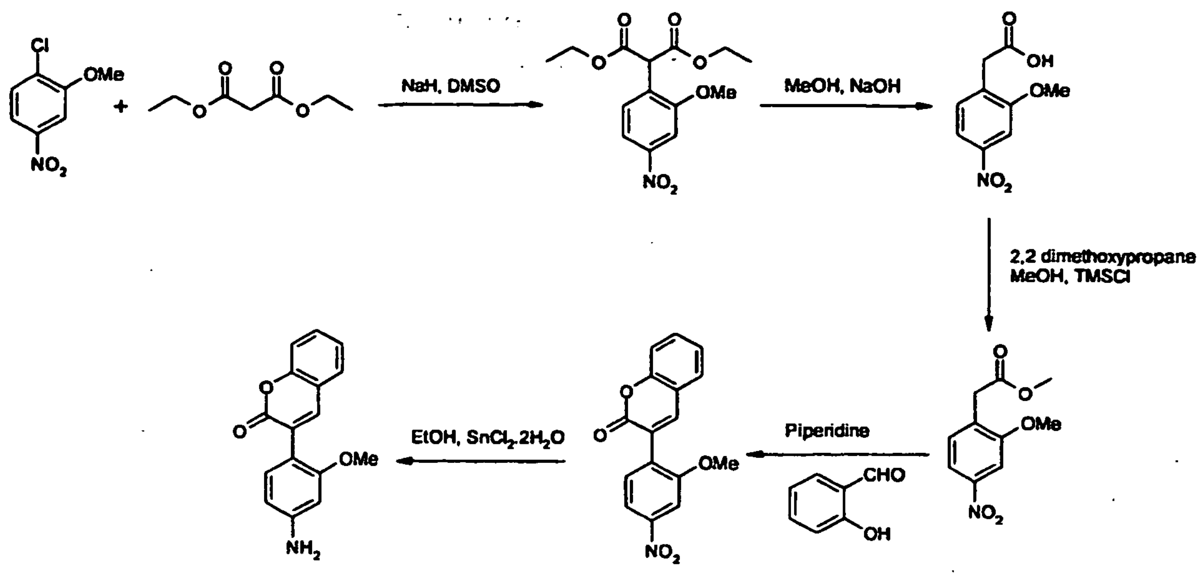


Step 1: Preparation of 2-(2-methoxy-d-nitro-phenyl)-malonic acid diethyl ester

Diethyl malonate (253 mmol) was slowly added at room temperature to a suspension of NaH (60% in oil, washed with cyclohexane) (275 mmol) in DMSO (140 ml). The reaction was stirred for 20 minutes before addition of 2-chloro-5-nitroanisole (105.5 mmol). After 5 hours of stirring at 110˚C, the solution was poured onto water (1.5 1) then acidified to pH=1 by addition of concentrated HCl. The solution was stirred for another 20 minutes. The reaction mixture was filtered and the solid obtained, washed with water and dried yielding 27.1 g of a red solid.

The residue was triturated in ice-cold methanol, filtered, washed with cold methanol and dried to yield 19.4g of the desired product.

Step 2: Preparation of (2-methoxy-4-nitro-phenyl)-acetic acid

The substrate (62.3 mmol) (prepared in Step 1) was dissolved in methanol (50 ml) and cooled with an ice bath. To this solution was added a solution of 6M NaOH (31 ml) during a 15 minute period and the resulting mixture was stirred for an additional 5 minutes at 0˚C. The reaction mixture was then warmed to 50˚C and stirred at this temperature for 90 minutes.

The methanol was removed under reduced pressure. The residue was cooled to 0˚C and acidified to pH=1 by addition of concentrated HCl. The reaction mixture was filtered and the solid washed with water. Ethyl acetate was added to the filtrate and the biphasic solution extracted. The organic layer was dried over magnesium sulfate, filtered and evaporated under reduced pressure to yield 9.4g of the desired product.

Step 3: Preparation of (2-methoxy-4-nitro-phenyl)-acetic acid methyl ester

The substrate (39.3 mmol) (prepared in Step 2) was dissolved in a mixture of methanol (45 ml) and 2,2-dimethoxypropane (16 ml). Chlorotrimethylsilane (0.5 ml) was added and the solution was stirred at room temperature for 2 hours.

The solvents were removed under reduced pressure. The residue was dissolved in ethyl acetate, washed with $NaHCO_3$, water and saturated aqueous sodium chloride. The organic phase was dried over magnesium sulfate, filtered and evaporated under reduced pressure to yield 8.4g of an orange solid.

Step 4: Preparation of 3-(2-methoxy-4-nitro-phenyl)-chromen-2-one

Salicylaldehyde (29 mmol) was added at room temperature to a solution of substrate (19.5 mmol) (prepared in Step 3) in piperidine (20 ml). The reaction mixture was stirred under reflux conditions for 4 hours. At the end of the reaction, the solution is cooled to 0˚C and ethanol was added. The precipitate was filtered and washed with ethanol to yield 3.6 g of the title compound as a yellow powder.

Step 5: Preparation of 3-(4-amino-2-methoxy-phenyl)-chromen-2-one

To a solution of substrate (12.2 mmol) (prepared in Step 4) in ethanol (40 ml) was added tin (II) chloride dihydrate (61 mmol). The resulting reaction mixture was stirred under reflux conditions for 2.5 hours. The solution was allowed to cool to room temperature before addition of NaOH 1M and stirred for 1 hour.

The mixture was filtered on celite and washed with ethanol. The ethanol was removed under reduced pressure and the resulting solution extracted with ethyl acetate (3x). The organic layers were combined and washed with NaOH 1M, water and saturated aqueous sodium chloride. The organic phase was dried over magnesium sulfate, filtered and evaporated under reduced pressure to yield 2.8g of a yellow powder.

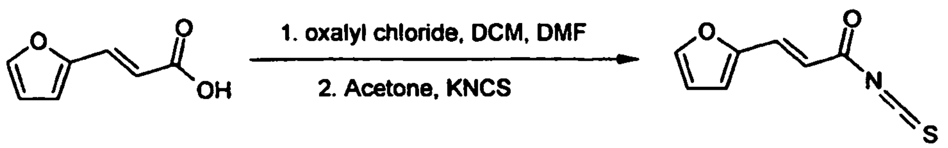


Stop 6: Preparation of (E)-3-furan-2-yl-acryloyl isothiocyanate

3-(2-furyl)acrylic acid (obtained from Aldrich; 72.4 mmol) was dissolved in DCM (50ml). To this solution were successively added oxalyl chloride (145 mmol) and DMF (0.5 ml). The reaction was stirred at room temperature for 1 hour. The solvents were evaporated under reduced pressure and azeotroped with toluene. The residue was dissolved in acetone (50 ml). To this solution was added potassium thiocyanate (145 mmol) and the solution was stirred for an additional 30 minutes. The solvents were evaporated and the residue was purified by flash column chromatography (100% DCM) to yield 10.6 g of yellow oil.

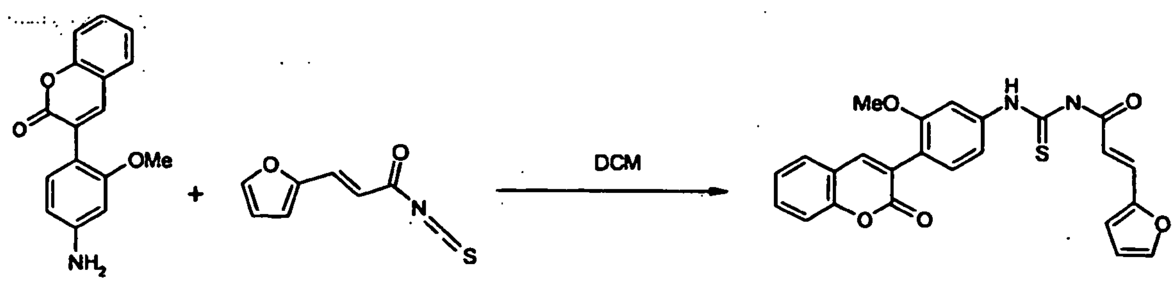


**1.A.1:** Preparation of (2*E*)-3-(2-furyl)-*N*-({[3-methoxy-4-(2-oxo-2H-chromen-3-yl)phenyl]amino} carbonothioyl) acrylamide (Compound 01).

(Alternative name: 1-((E)-3-furan-2-yl-acryloyl)-3-[3-methoxy-4-(2-oxo-2H-chromen-3-yl)-phenyl]-thiourea)

3-(4-amino-2-methoxy-phenyl)-chromen-2-one (6 mmol) (prepared in Step 5) and (E)-3-furan-2-yl-acryloyl isothiocyanate (24 mmol) (prepared in Step 6) were dissolved in DCM (50 ml). The reaction was stirred at room temperature for 2 hours. The solvents were evaporated under reduced pressure. The residue was dissolved in ethanol and the solution was stirred for an additional 30 minutes. The solution was filtered and the solid washed to yield 2.6 g of a yellow powder (mp 227-230 °C). LCMS ($M+H^+$= 447, 100%), NMR (250 MHz, DMSO): δ 12.89 (br s, 1H), 11.72 (br s, 1H), 8.07 (s, 1H), 7.93 (s, 1H), 7.80-7.30 (m, 8H), 7.01 (d, J 3.5, 1H), 6.82 (d, J 15.5, 1H), 6.70-6.67 (m, 1H), 3.78 (s, 3H).

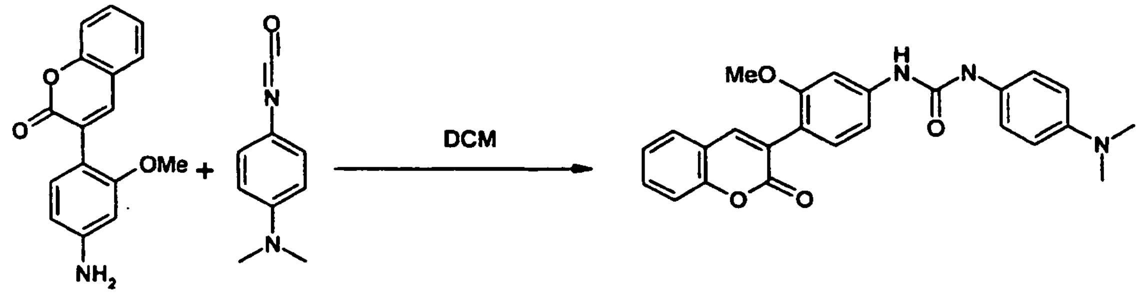


1.A.2: Preparation of 1-(4-dimethylamino-phenyl)-3-[3-methoxy-4-(2-oxo-2H-chromen-3-yl)-phenyl]-urea hydrochloride salt (Compound 06 hydrochloride salt).

3-(4-amino-2-methoxy-phenyl)-chromen-2-one (12.8 mmol) (prepared in Step 5) and 4-(Dimethylamino)phenyl isocyanate (15.4 mmol) were dissolved in DCM (35 ml). The reaction was stirred at room temperature for 6 days. The solvents were evaporated under reduced pressure. The residue was triturated with diethyl ether, filtered and the solid washed with diethyl ether to yield 5.6 g of solid. This solid was dissolved in methanol. A solution of hydrogen chloride in methanol was added ant the resulting mixture was stirred for 20 minutes. The solvents were evaporated under vacuum. The residue obtained was washed with diethyl ether to yield 5.7 g of a light beige powder. LCMS ($M+H^+$= 430, 100%), NMR (250 MHz, DMSO): δ 9.43-9.30 (m, 2H), 8.00 (s, 1H), 7.73 (d, J 7.8, 1H), 7.65-7.30 (m, 8H), 7.25 (d, J 8.0, 1H), 7.01 (dd, J 8.2 and 1.7, 1H), 3.74 (s, 3H), 3.16 (s, 6H).

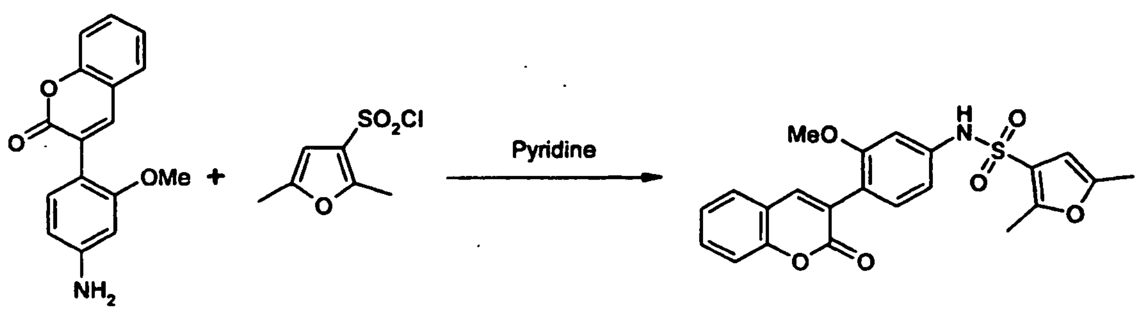


1.A.3: Preparation of *N*-[3-methoxy-4-(2-oxo-2*H*-chromen-3-yl)phenyl]-2,5-dimethylfuran-3-sulfonamide (Compound 13).

(Alternative name: 2,5-dimethyl-furan-3-sulfonic acid-3-[3-methoxy-4-(2-oxo-1,2-dihydroquinolin-3-yl)-phenyl]-amide)

3-(4-amino-2-methoxy-phenyl)-chromen-2-one (5.6 mmol) (prepared in Step 5) and 2,5-dimethyl-furan-3-sulfonyl chloride (obtained by Maybridge; 6.2 mmol) were dissolved in pyridine (10 ml). The reaction mixture was stirred at room temperature for 12 hours. The solution was poured onto a solution of HCl 1M, extracted with DCM and washed with a solution of HCl 1M, water and a saturated solution of aqueous sodium chloride. The solvents were evaporated under reduced pressure. The residue was triturated with diethyl ether, filtered and washed with diethyl ether to yield 1.7 g of a beige powder. LCMS ($M+H^+$= 426, 100%), NMR (250 MHz, DMSO): δ 10.41 (br s, 1H), 7.98 (br s, 1H), 7.71 (dd, J 7.7 and 1.2, 1H), 7.61 (td, J 8.2 and 1.5, 1H), 7.45-7.25 (m, 2H), 7.23 (d, J 8.2, 1H), 6.85 (d, J 1.7, 1H), 6.75 (dd, J 8.2 and 2, 1H), 6.26 (s, 1H), 3.68 (s, 3H), 2.41 (s, 3H), 2.21 (s, 3H).

**1.B** Synthesis of oxoquinoline derivatives.

$^1$R and $^4$R are potential substituents of cycles (I), (II) and (III) in formula (a), (a'), (b) and (c), as defined above. $^5$R represents potential substituents in order to obtain compounds according to formula (a), (a'), (b) or (c) as defined above.

The main steps and conditions to be applied are summarized in the following reaction scheme:

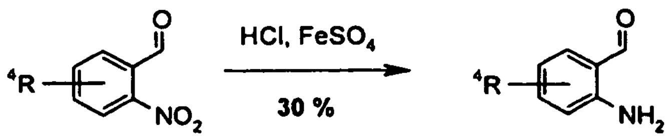


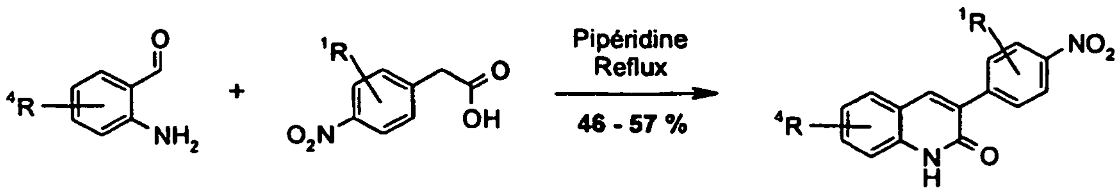


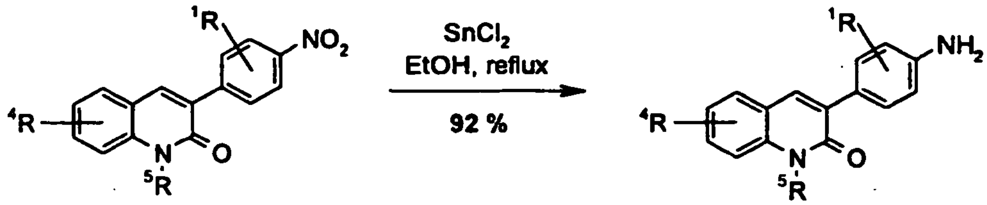


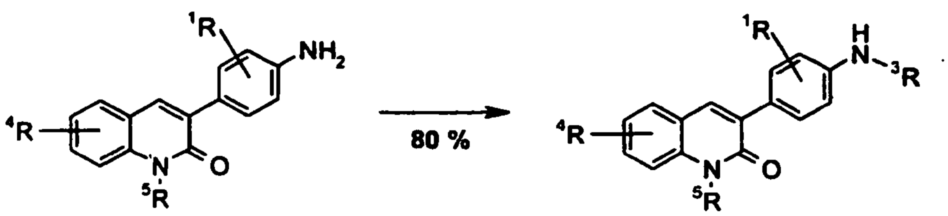


More specifically:

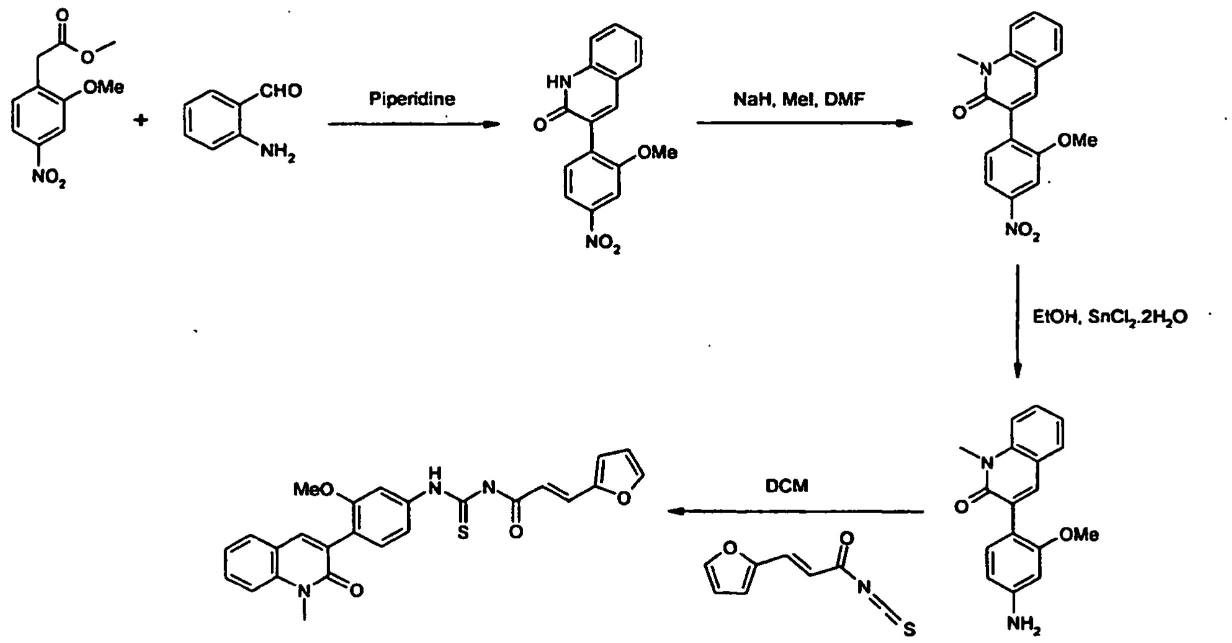


Step 1: Preparation of 3-(2-methoxy-4-nitro-phenyl)-1H-quinolin-2-one

o-aminobenzaldehyde (prepared according to procedure described in Org. Synth., coll. Vol. 3, 56) (17 mmol) was added at room temperature to a solution of (2-methoxy-4-nitro-phenyl)-acetic acid methyl ester (15 mmol) (prepared in Step 3 of Example 1A) in piperidine (15 ml). The reaction mixture was stirred under reflux conditions for 2 hours. At the end of the reaction, the solution was cooled to room temperature and ethanol (50 ml) was added. The mixture was stirred for 30 minutes then cooled to 0°C. The precipitate was filtered to yield 2.2 g of the title compound as a yellow solid.

Step 2: Preparation of 3-(2-methoxy-4-nitro-phenyl)-1-methyl-1H-quinolin-2-one

The substrate (7.4 mmol) (prepared in Step 1) was slowly added at 0°C to a suspension of NaH (60% in oil, washed with cyclohexane) (14.8 mmol) in DMF (20 ml). The reaction was stirred for 5 minutes at 0°C before addition of methyl iodide (11.1 mmol). After 1 hours of stirring at 0°C. the solution was poured onto water (150 ml) and filtered. The residue was dissolved in EtOAc and dried over anhydrous magnesium sulfate. The solvent was removed under reduced pressure to yield 2.1 g of the desired product as a yellow solid.

Step 3: Preparation of 3-(4-amino-2-methoxy-phenyl)-1-methyl-1H-quinolin-2-one

To a solution of substrate (6.8 mmol) (prepared in Step 2) in ethanol (100 ml) was added tin (II) chloride dihydrate (33.8 mmol). The resulting reaction mixture was stirred under reflux conditions for 2 hours. The solvents were removed under reduced pressure and the residue obtained, dissolved in ethyl acetate and washed with a saturated solution of aqueous sodium hydrogen carbonate and a saturated solution of aqueous sodium chloride.

The organic phase was dried over magnesium sulfate, filtered and evaporated under reduced pressure to yield 1.9 g of a yellow solid.

**1B.1:** Preparation of (2*E*)-3-(2-furyl)-*N*-({[3-methoxy-4-(1-methyl-2-oxo-1,2-dihydroquinolin-3-yl)phenyl]amino}carbonothioyl)acrylamide (Compound 17).

(Alternative name:. 1-(E)-3-furan-2-yl-acryloyl)-3-[3-methoxy-4-(1-methyl-2-oxo-1,2-dihydroquinolin-3-yl)-phenyl]-thiourea)

3-(4-amino-2-methoxy-phenyl)-1-methyl-1H-quinolin-2-one (6.8 mmol) (prepared in Step 3) and (E)-3-furan-2-yl-acryloyl isothiocyanate (13.7 mmol) (prepared in Step 6 of Example 1A) were dissolved in DCM (5 ml). The reaction was stirred at room temperature for 2 hours. The solvents were evaporated under reduced pressure. The residue was dissolved in ethanol and the solution was filtered to yield 2.2 g of a yellow solid. LCMS ($M+H^+$= 460, 99%). NMR (250

MHz, DMSO): δ 12.88 (br s, 1H), 11.71 (br s, 1H), 7.95-7.85 (m, 2H), 7.74 (d, J 7.7, 1H), 7.70-7.50 (m, 4H), 7.28-7.20 (m, 3H), 7.01 (d, J 3.5, 1H), 6.82 (d, J 15.5, 1H), 6.70-6.68 (m, 1H), 3.73 (s, 3H), 3.68 (s, 3H).

**1.C:** Methylation conditions

Compound 14 was formed by the reaction of the compound formed in example 1.A.1 (compound 01) with $K_2CO_3$ (2eq.), MeI (1.5 eq), in Acetone. The reaction was heated to reflux for 4hours.

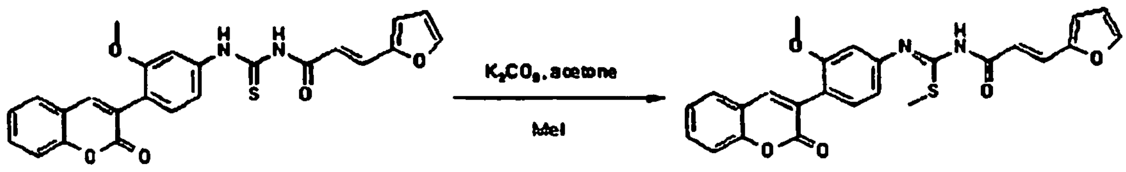


**1.D**

The characterization of representative compounds of the invention, obtained following the procedures of Examples 1.A, 1.B and 1.C and appropriate variations thereof, are reported below.
The LC_MS (Liquid Chromatography Mass Spectrometry) details were the following:

Automatic injector with an injection volume of 10μl
Column: THERMO HYPERSIL HYPERPREP RP C18, 8μm, 150*4.6mm
UV Detector: Agilent G1315A diode array detector [190-400nm]
ELSD (evaporative Light Scattering Detection) detector: Polymerlabs ELS2100, T˚ nebulizer = 50˚C, T˚ evaporator =90˚C, gaz flow rate: 1.6 SLM

**Solvent conditions**

| Time (min) | % (Water +0.05% TFA) | %(ACN + 0.05%TFA) | Flow rate (ml/min) |
|---|---|---|---|
| 0 | 80 | 20 | 1 |
| 8 | 0 | 100 | 1 |
| 8.10 | 0 | 100 | 1 |
| 13 | 0 | 100 | 1 |
| Column regeneration time : 7min | | | |

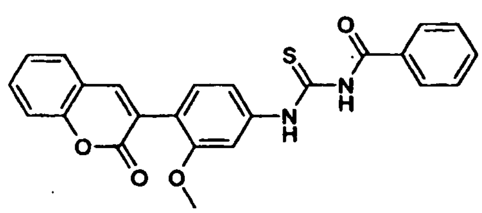

Compound 02: *N*-({[3-methoxy-4-(2-oxo-2*H*-chromen-3-yl)phenyl]amino}carbonothioyl) benzamide. (alternative name : 1-Benzoyl-3-[3-methoxy-4-(2-oxo-2H-chromen-3-yl)-phenyl]-thiourea) NMR (250 MHz, DMSO): δ 12.75 (s, 1H), 11.65 (s, 1H), 8.08 (s, 1H), 8.02-7.95 (m, 2H), 7.80-7.35 (m, 10H), 3.78 (s, 3H).
LCMS 98% (431, M+1)

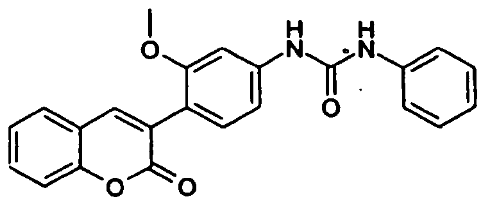

Compound 03: 1-[3-methoxy-4-(2-oxo-2H-chromen-3-yl)-phenyl]-3-phenyl-urea:
Pale yellow solid.
NMR (250 MHz, DMSO): δ 8.88 (s, 1H), 8.74 (s, 1H), 8.00 (s, 1H), 7.73 (d, J 7.7, 1H), 7.61 (t, J 8.5, 1H), 7.52-7.40 (m, 8H), 7.15-6.85 (m, 2H), 3.75 (s, 3H).
LC-MS purity: 100%.

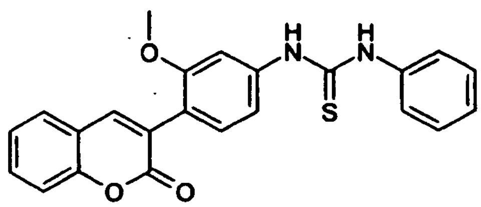

Compound 04: 1-[3-methoxy-4-(2-oxo-2H-chromen-3-yl)-phenyl]-3-phenyl-thiourea:
White solid.
NMR (250 MHz, DMSO): δ 9.94 (br s, 2H), 8.02 (s, 1H), 7.74 (dd, J 6.2 and 1.2, 1H), 7.62 (td, J 7.0 and 1.2, 1H), 7.50 (d, J 6.2, 2H); 7.45-7.25 (m, 6H), 7.20-7.10 (m, 2H), 3.74 (s, 3H). LC-MS purity: 100%.

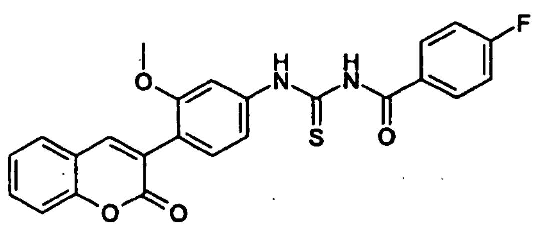

Compound 05: 4-fluoro-*N*-({[3-methoxy-4-(2-oxo-2*H*-chromen-3-yl)phenyl]amino} carbonothioyl)benzamide. (alternative name: 1-(4-fluoro-benzoyl)-3-[3-methoxy-4-(2-oxo-2H-chromen-3-yl)-phenyl]-thiourea)
White solid.
NMR (250 MHz, DMSO): δ 12.66 (br s, 1H), 11.70 (br s, 1H), 8.15-8.05 (m, 3H), 7.75 (d, J 8.2, 1H), 7.70-7.60 (m, 2H), 7.50-7.35 (m, 6H), 3.77 (s, 3H).
LC-MS purity: 100%.

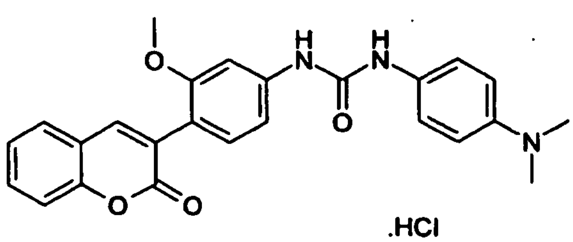

Compound 06: 1-(4-dimethylamino-phenyl)-3-[3-methoxy-4-(2-oxo-2H-chromen-3-yl)-phenyl]-urea.HCl:
Pale yellow solid.
NMR (250 MHz, DMSO): δ 9.43-9.30 (m, 2H), 8.00 (s, 1H), 7.73 (d, J 7.8, 1H), 7.65-7.30 (m, 8H), 7.25 (d, J 8.0. 1H),

7.01 (dd, J 8.2 and 1.7, 1H), 3.74 (s, 3H), 3.16 (s, 6H).
LC-MS purity: 98%

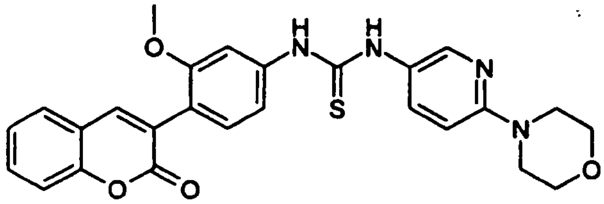

Compound 08: 1-[3-methoxy-4-(2-oxo-2H-chromen-3-yl)-phenyl]-3-(6-morpholin-4-yl-pyridin-3-yl)-thiourea:

Yellow solid.
NMR (250 MHz, DMSO): δ 9.87 (s, 1H), 9.62 (s, 1H), 8.10 (d, J 2.7, 1H), 8.01 (s, 1H), 7.75 (dd, J 8.2 and 0.7, 1H), 7.70-7.55 (m, 2H), 7.45-7.25 (m, 4H), 7.12 (dd, J 8.0 and 1.5, 1H), 6.83 (d, J 8.7, 1H), 3.80-3.65 (m, 7H), 3.45-3.35 (m, 4H).
LC-MS purity: 92%

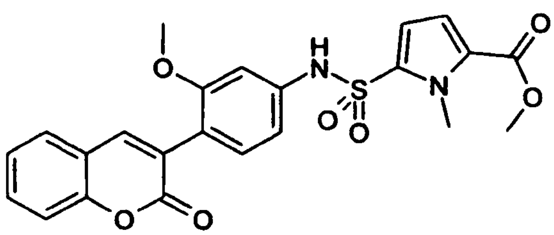

Compound 09: methyl 5-({[3-methoxy-4-(2-oxo-2*H*-chromen-3-yl)phenyl]amino}sulfonyl)-1-methyl-1 *H*-pyrrole-2-carboxylate: (alternative name: 5-[3-methoxy-4-2-oxo-2H-chromen-3-yl)-phenylsulfamoyl]-1-methyl-1H-pyrrole-2-carboxylic acid methyl ester).
Yellow solid
NMR (250 MHz, DMSO): δ 10.30 (br s, 1H), 7.96 (s, 1H), 7.78 (d, J 2.0, 1H), 7.70 (dd, J 8.0 and 1.5, 1H), 7.60 (td, J 8.2 and 1.5, 1H), 7.45-7.30 (m, 2H), 7.21 (d, J 8.2, 1H), 7.03 (d, J 2.0, 1H), 6.86 (d, J 2.0, 1H), 6.77 (dd, J 8.2 and 2.0, 1H), 3.86 (s, 3H), 3.75 (s, 3H), 3.68 (s, 3H).
LC-MS purity: 96%

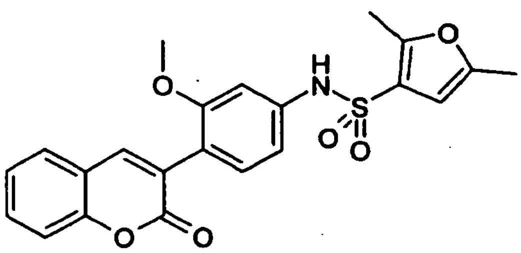

Compound 13: *N*-[3-methoxy-4-(2-oxo-2*H*-chromen-3-yl)phenyl]-2,5-dimethylfuran-3-sulfonamide. (Alternative name: 2,5-dimethyl-furan-3-sulfonic acid [3-methoxy-4-(2-oxo-2H-chromen-3-yl)-phenyl]-amide)
Off-white solid.
NMR (250 MHz, DMSO): δ 10.41 (s, 1H), 7.98 (s, 1H), 7.71 (d, J 7.5, 1H), 7.61 (t, J 8.0, 1H), 7.43-7.33 (m, 2H), 7.23 (d, J 8.0, 1H). 6.84 (s, 1H), 6.75 (d, J 8.2, 1H), 6.26 (s, 1H), 3.68 (s, 3H), 2.41 (s, 3H), 2.21 (s, 3H).
LC-MS purity: 97%

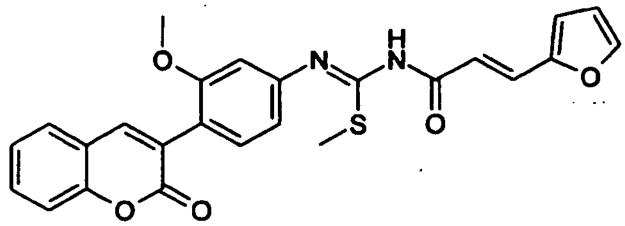

Compound 14: methyl *N*-[(2*E*)-3-(2-furyl)prop-2-enoyl]-*N*-[3-methoxy-4-(2-oxo-2*H*-chromen-3-yl)phenyl]imidothiocarbamate (Alternative name: 1-((E)-3-furan-2-yl-acryloyl)-3-[3-methoxy-4-(2-oxo-2H-chromen-3-yl)-phenyl]-2-methyl-isothiourea) :
Yellow solid.
NMR (250 MHz, CDCl3): δ 7.79 (s, 1H), 7.70-7.25 (m, 7H), 7.05-6.85 (m, 2H), 6.75-6.45 (m, 3H), 3.83 (s, 3H), 2.57 (br s, 3H).
LC-MS purity: 96%

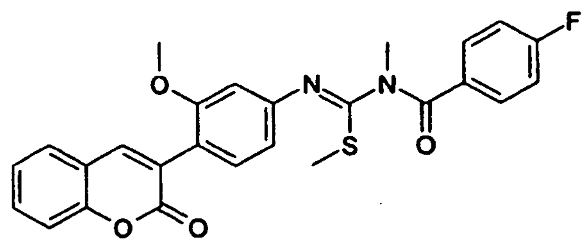

Compound 25: 1-(4-fluoro-benzoyl)-3-[3-methoxy-4-(2-oxo-2H-chromen-3-yl)-phenyl]-1,2-dimethyl-isothiourea:
White solid.
NMR (250 MHz, DMSO): δ 8.20-8.05 (m, 3H), 7.85-7.60 (m, 2H), 7.55-7.20 (m, 6H), 7.15-7.05 (m, 1H), 3.78 (s, 3H), 3.45 (s, 3H), 2.19 (s, 3H).
LC-MS purity: 97%

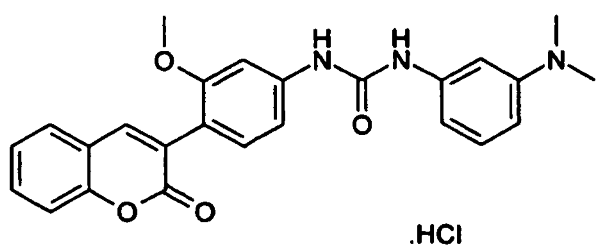

Compound 18: 1-(3-dimethylamino-phenyl)-3-[3-methoxy-4-(2-oxo-2H-chromen-3-yl)-phenyl]-urea.HCl:
Off-white solid.
NMR (250 MHz, DMSO): δ 8.85 (br s, 1H), 8.65 (br s, 1H), 8.00 (s, 1H), 7.74 (dd, J 7.5 and 0.75, 1H), 7.61 (td; J 7.9 and 1.5, 1H), 7.45-6.95 (m, 7H), 6.90-6.75 (m, 1H), 6.50-6.35 (m, 1H), 3.75 (s, 3H), 2.91 (s, 6H).
LC-MS parity: 99%

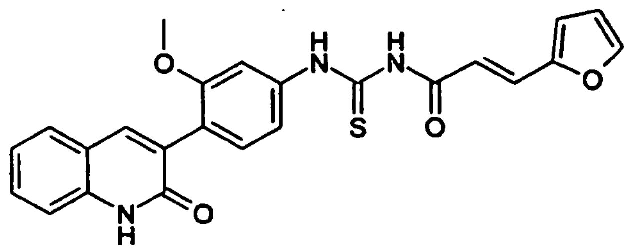

Compound 15: (2*E*)-3-(2-furyl)-*N*-({[3-methoxy-4-(2-oxo-1,2-dihydroquinolin-3-yl)phenyl] amino}carbonothioyl)acrylamide (Alternative name : 1-((E)-3-furan-2-yl-acryloyl)-3-[3-methoxy-4-(2-oxo-1,2-dihydro-quinolin-3-yl)-phenyl]-thiourea) :
Yellow solid.
NMR (250 MHz, DMSO): δ 12.87 (s, 1H), 11.84 (s, 1H), 11.70 (s, 1H), 8.00-7.85 (m, 2H), 7.75-7.22 (m, 7H), 7.18 (t, J 7.5, 1H), 7.01 (d, J 3.2, 1H), 6.81 (d, J 15.5, 1H), 6.70-6.66 (m, 1H), 3.74 (s, 3H).
LC-MS purity: 92%

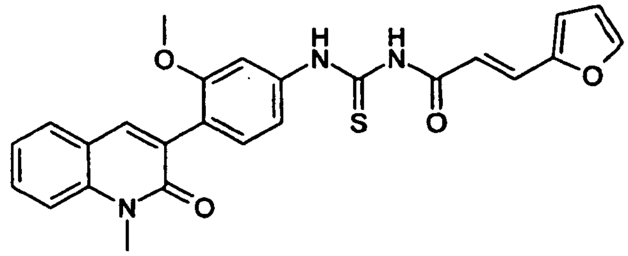

Compound 17 : (2*E*)-3-(2-furyl)-*N*-({[3-methoxy-4-(1-methyl-2-oxo-1,2-dihydroquinolin-3-yl) phenyl]amino}carbonothioyl)acrylamide.
(Alternative name: 1-((E)-3-furan-2-yl-acryloyl)-3-[3-methoxy-4-(1-methyl-2-oxo-1,2-dihydroquinolin-3-yl)-phenyl]-thiourea) :
Yellow solid.
NMR (250 MHz, DMSO): δ 12.88 (br s, 1H), 11.71 (br s, 1H), 7.95-7.85 (m, 2H), 7.74 (d, J 7.7, 1H), 7.70-7.50 (m, 4H), 7.28-7.20 (m, 3H), 7.01 (d, J 3.5, 1H), 6.82 (d, J 15.5, 1H), 6.70-6.68 (m, 1H), 3.73 (s, 3H), 3.68 (s, 3H).
LC-MS purity: 93%

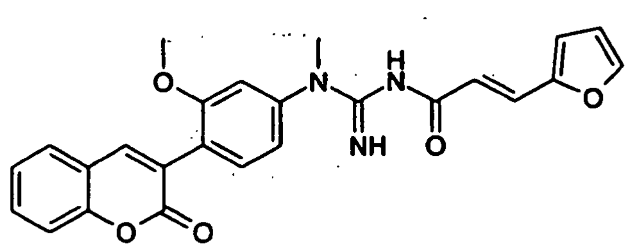

Compound 19: N'-((E)-3-furan-2-yl-acryloyl)-N-[3-methoxy-4-(2-oxo-2H-chromen-3-yl)-phenyl]-N-methyl-guanidine:
Off-white solid.
NMR (250 MHz, CDCl3): δ 8.06 (br s, 1H), 7.85-6.95 (m, 9H), 6.80-6.75 (m, 1H), 6.60-6.55 (m, 1H), 6.31 (d, J 15.5, 1H), 3.79 (br s, 3H), 3.56 (br s, 3H).
LC-MS purity: 99%

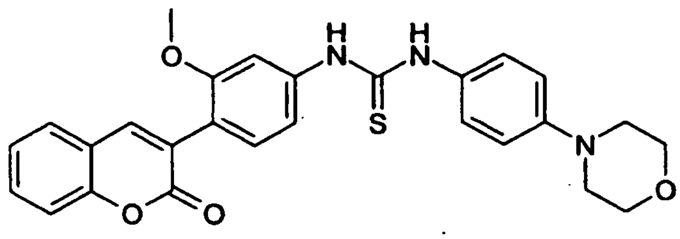

Compound 20: 1-[3-methoxy-4-(2-oxo-2H-chromen-3-yl)-phenyl]-3-(4-morpholin-4-yl-phenyl)-thiourea:
Yellow solid.
NMR (250 MHz, DMSO): δ 9.70 (br s, 2H), 7.99 (s, 1H), 7.73 (d, J 7.7, 1H), 7.61 (t, J 7.2, 1H), 7.45-7.20 (m, 6H), 7.10 (dd, J 8.5 and 1.7, 1H), 6.91 (d, J 8.7, 2H), 3.75-3.65 (m, 7H), 3.10-3.3.05 (m, 4H).
LC-MS purity: 93%

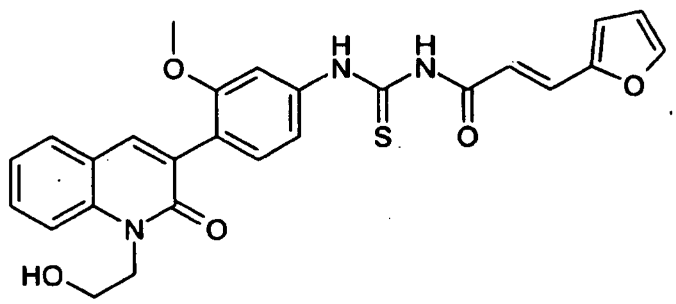

Compound 21 : 1-((E)-3-furan-2-yl-acryloyl)-3-{4-[1-2-hydroxy-ethyl)-2-oxo-1,2-dihydro-quinolin-3-yl]-3-methoxy-phenyl}-thiourea :
Pale yellow solid.
NMR (250 MHz, DMSO): δ 12.87 (br s, 1H), 11.70 (br s, 1H), 7.95-7.85 (m, 2H), 7.90-7.55 (m, 5H), 7.20-7.10 (m, 3H), 7.01 (d, J 3.5, 1H), 6.82 (d, J 15.7, 1H), 6.70-6.60 (m, 1H), 4.95 (t, J 5.2, 1H), 4.37 (t, J 6.5, 2H), 3.80-3.60 (m, 5H).
LC-MS purity: 97%

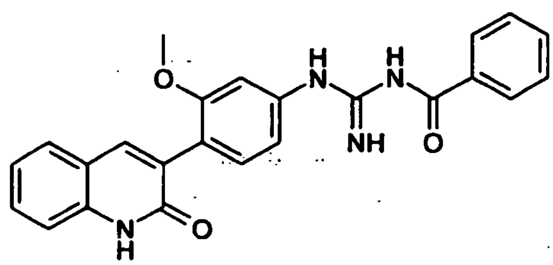

Compound 22: N-Benzoyl-N'-[3-methoxy-4-(2-oxo-1,2-dihydro-quinolin-3-yl)-phenyl]-guanidine :
White solid.
NMR (250 MHz, DMSO): δ 11.82 (s, 1H), 9.40 (br s, 1H), 8.17-8.12 (m, 2H), 7.87 (s, 1H), 7.66 (d, J 8.0, 1H), 7.60-7.25 (m, 8H), 7.20 (t, J 7.5, 1H), 7.02 (d, J 6.7, 1H), 3.77 (s, 3H).
LC-MS purity: 93%

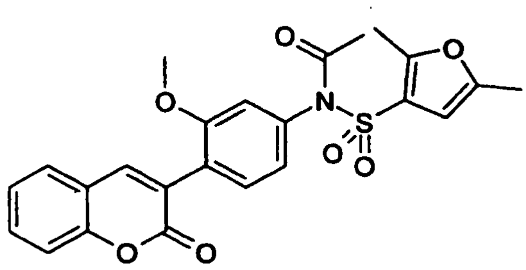

Compound 23: 2,5-dimethyl-furan-3-sulfonic acid acetyl-[3-methoxy-4-(2-oxo-2H-chromen-3-yl)-phenyl]-amide:
White foam.
NMR (250 MHz, CDCl3): δ 7.83 (s, 1H), 7.65-7.45 (m, 3H), 7.45-7.20 (m, 2H), 6.95-6.70 (m, 2H), 6.26 (s, 1H), 3.85 (s, 3H), 2.57 (s, 3H), 2.29 (s, 3H), 2.01 (s, 3H).
LC-MS purity: 86%

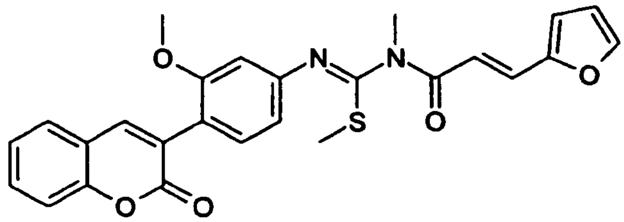

Compound 24: 1-((E)-3-furan-2-yl-acryloyl)-3-[3-methoxy-4-(2-oxo-2H-chromen-3-yl)-phenyl]-1,2-dimethyl-isothiourea:
Yellow foam.
NMR (250 MHz, DMSO): δ 8.02 (br s, 1H), 7.85 (br s, 1H), 7.72 (d, J 8.2, 1H), 7.61 (t, J 7.5, 1H), 7.45-7.20 (m, 4H), 6.92 (br s, 1H), 6.70-6.35 (m, 4H), 3.55 (br s, 3H), 3.05 (br s, 3H), 2.55 (br s, 3H).
LC-MS purity: 98%

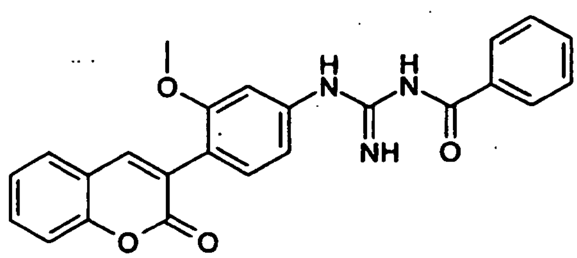

Compound 07 : *N*-(imino{[3-methoxy-4-(2-oxo-2*H*-chromen-3-yl)phenyl]amino}methyl) benzamide.
(Alternative name : N-Benzoyl-N'-[3-methoxy-4-(2-oxo-2H-chromen-3-yl)-phenyl]-guanidine) White solid.
NMR (250 MHz, CDCl3): δ 8.17-8.12 (m, 2H), 7.74 (s, 1H), 7.60-7.20 (m, 8H), 6.85-6.75 (m, 2H), 3.73 (s, 3H).
LC-MS purity: 100%

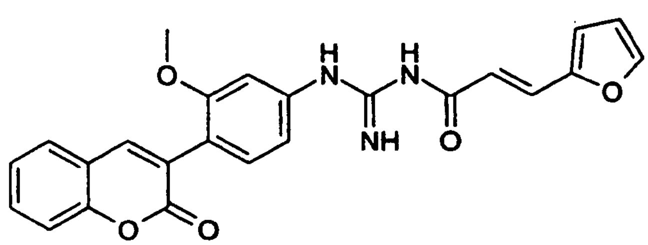

Compound 12 : (2*E*)-3-(2-furyl)-*N*-(imino{[3-methoxy-4-(2-oxo-2*H*-chromen-3-yl)phenyl] amino}methyl)acrylamide:
(Alternative name: N-((E)-3-furan-2-yl-acryloyl)-N'-[3-methoxy-4-(2-oxo-2H-chromen-3-yl)-phenyl]-guanidine)
White solid.
NMR (250 MHz, DMSO): δ 11.82 (br s, 1H), 10.93 (br s, 1H), 9.13 (br s, 1H), 8.03 (s, 1H), 7.96 (s, 1H), 7.79 (d, J 7.7, 1H), 7.70-7.60 (m, 2H), 7.55-7.35 (m, 3H), 7.19 (d, J 1.0, 1H), 7.15-7.00 (m, 2H), 6.80-6.60 (m, 2H), 3.79 (br s, 3H).
LC-MS purity: 95%

**Example 2:** Test for proliferation / survival *in vitro* and determination of cell cycle.

2A *Propidium iodide staining (assay for cytotoxic effect)*

Propidium iodide (PI) cannot normally cross the cell membrane; it is thus excluded by viable cells but not from dying or dead cells, having a damaged membrane, where it intercalates into double-stranded nucleic acids and fluoresces when excited at 488 nm.

In order to determine the cytotoxic effect of a compound, $2.10^5$ cells are incubated with the compound to be tested during a time ranging from hours to days, then the cells are collected either by simple pipeting if the cells grow in suspension or after mild trypsinization for adherent cells. The cells are pelleted by gentle centrifugation 1200rpm/ 5min/4˚C, then the supernatant is discarded and replaced by 1mL of fresh medium. One hundred microliters of propidium iodide (40μg/mL) are added to 400μL of the cell suspension in a FACS tube and viability is measured by flow cytometry as the percentage of cells whose fluorescence recorded on FL-3 channel is comprised between 0 and 2.

2B. Cell cycle analysis

In order to determine the cytostatic effect of a compound, $2.10^5$ cells are incubated with the compound to be tested during a time ranging from hours to days, then the cells are collected either by simple pipeting if the cells grow in suspension or after mild trypsinization for adherent cells. The cells are pelleted by gentle centrifugation 1200rpm/ 5min/4˚C, then the supernatant is discarded and the cells are fixed with 1mL of PBS / Ethanol 70% overnight at -20˚C. The tubes are then centrifuged during 5 minutes at 1200rpm and the supernatant is removed. The pellet is then resuspended in 450μL of PBS before 50μL of RNAse (1mg/mL) and 5μL of propidium iodide (2mg/mL) are added. Each tube is left 30 minutes at 37˚C and immediately analysed by flow cytometry after incubation.

A biparametric dot plot representation FL3 peak vs FL3 integer is plotted and the cells located on the diagonal are gated and analysed on a histogram representing the number of cells as a function of the FL3 peak.

To assess the relative position of cursors defining relative proportion of cells in subG1, G1, S or G2 phase of the cell cycle, G1 and G2 peaks are to be defined first:

The G1 peak is defined as the major peak in absence of treatment (generally X=200);
The G2 peak is centered on the value which is twice the value of the G1 peak (generally X=400).

Cells in subG1 phase are plotted in the part of the histogram preceding the G1 peak (generally X<200) and cells in S phase are enclosed in the area between the G1 and G2 peaks.

On this graphic, subG1, G1, S and G2/M phases of the cell cycle can thus be defined.

2*C Proliferation test* ViaLight® HS High Sensitivity Cytotoxicity and Cell Proliferation BioAssay Kit

This protocol allows the determination of the cytostatic and cytotoxic activities of compounds to be tested on mammalian cells in culture and was carried out essentially according to the manufacturer's recommendations.

Cells are suspended in 15mL of their respective media and are enumerated. They are then diluted in their media in order to obtain:

- 2500 cells per 90 μL per well for MRC5 cells
- 5000 cells per 90 μL per well for all other cell lines.

Cells are then seeded in a 96 well plate, and incubated at least 4 hours in the incubator at 37˚C in 5% $CO_2$.

The molecules to be tested are diluted in DMEM with DMSO in order to obtain the following dilutions:

- dilution at 250μM / 1% DMSO
- dilution at 50μM / 1% DMSO

- dilution at 10µM / 1% DMSO
- dilution at 2µM / 1% DMSO
- dilution at 400nM / 1 % DMSO
- dilution at 80nM / 1% DMSO

For each well comprising 90µL of cell suspension, 10µL of a solution of the compound to be tested is added, with the 6 different concentrations. The plate is then incubated during 72 hours. One control plate is contacted with 10µL of DMEM 1% DMSO, without compound to be tested. 50µL of lysis buffer (test vialight® lysis buffer) is added in each well of the control plate and the plate is incubated 10 minutes under hood.
Then, 100µL of test buffer (test vialight® test buffer) is added in each well and the control plate is incubated during 2 minutes. The luminescence is read in each well. The mean luminescence for each type of cells is called T0.
After the 72h incubation, in order to determine the viability, 50µL of lysis buffer (test vialight® lysis buffer) is added in each well of the plates and the plates are incubated 10 minutes under hood.
Then, 100µL of test buffer (test vialight® test buffer) is added in each well and the plates are incubated during 2 minutes. The luminescence is read in each well. For each type of cell, the mean luminescence in the well not containing the compound is called C72 and the mean luminescence in well containing a given concentration of a compound to be tested is called T72. For each dilution (concentration of compound to be tested), T0 and T72 values are compared.
If T72 is superior or equal to T0, the following formula is applied to determine the growth rate:

$$\mathbf{Y = Growth\ (\%) = (T72 - T0) / (C72 - T0) \times 100}$$

If T72 is inferior to T0, then the following formula is applied to determine the lethality rate :

$$\mathbf{Y = LC\ (\%) = (T72 - T0) / T0 \times 100}$$

The graph is plotted for the different concentrations of compound and

- GI50 (50% of growth inhibitory) is determined, this is the dose producing 50% of cell growth (intersection at Y=50);
- TGI (total growth inhibitory) is determined on the graph, this is the dose producing the total arrest of the cell growth (intersection at Y=0); and
- LC50 (50% of lethal concentration) is determined, this is the dose producing the death of 50% of the cells (intersection at Y= - 50).

**Example 3:** Inhibition of cell proliferation *in vitro* with compound (01).

The effect of a compound of the invention, compound (01): (2*E*)-3-(2-furyl)-*N*-({[3-methoxy-4-(2-oxo-2*H*-chromen-3-yl)phenyl]amino}carbonothioyl)acrylamide, on cell proliferation has been tested on different cells, i.e. MRC5 (normal non-transformed lung fibroblasts), H146 (small cell lung carcinoma), H69 (small cell lung carcinoma), H69AR (small cell lung carcinoma, adriamycin resistant), U937 (histiocytic lymphoma), BL41 (Burkitt Lymphoma), Val (lymphoma), RS4-11 (acute lymphoblastic leukaemia), H460 (non-small cell lung carcinoma), PC3 (prostate adenocarcinoma), MDA231 (breast epithelial adenocarcinoma), MCF7 (breast epithelial adenocarcinoma), Lovo (colorectal adenocarcinoma) and A375 (melanoma), at different concentrations. The protocol is detailed in example 2C.
The results are depicted in **fig.1A and 1B**.
A negative percentage of cell growth implies a cytotoxic effect, in addition to an antistatic effect In conclusion, it can be seen that compound (01) inhibits the growth of lung, prostate, breast, colon, melanoma, and myeloid tumor cells with IC50s in the range of 50nM to 1µM.
Viability and cell cycle profile, tested with compound (01), at 500nM:

| Cell line | Origin | Viability 48h | Viability 72h | Cell cycle profile - 72h |
|---|---|---|---|---|
| MRC5 | Non-transformed fibroblasts | 94% | 89% | G2/M accumulation |
| PC3 | Prostate | 92% | 90% | G2/M accumulation |
| NCI-H460 | Lung (NSCLC) | 80% | 80% | G2/M accumulation Strong sub-G1 |
| A375 | Melanoma | 65% | 35% | G2/M accumulation Strong sub-G1 |

(continued)

| Cell line | Origin | Viability 48h | Viability 72h | Cell cycle profile - 72h |
|---|---|---|---|---|
| U937 | Myeloid | 25% | 6% | Strong sub-G1 accumulation |
| CEM<br>Jurkat | Lymphoid<br>- | 10%<br>4% | 2%<br>1% | Strong sub-G1 accumulation |

In conclusion, it can be seen that the compound (01) ((2*E*)-3-(2-furyl)-*N*-({[3-methoxy-4-(2-oxo-2*H*-chromen-3-yl)phenyl]amino}carbonothioyl)acrylamide) has cytotoxic effect on lymphoid and myeloid tumor cells, with an apoptotic cell cycle profile and has cytostatic effect on prostate and lung tumor cells by cell cycle arrest at entry into mitosis.
It is also observed that control non-transformed cells (MRC5) are ur effected by contact with the molecule (01) in this experiment.

**Example 4:** Inhibition of tumor growth *in vivo.*

For this experiment, Swiss nude mice received a subcutaneous xenograft of $5x10^6$ human lung cancer NCI-H460 cells (NSCLC). The percentage of growth of the tumor size versus tumor size at the beginning of treatment is plotted during 15 days for mice receiving four different treatments (12 mice per group of treatment).
The first treatment corresponds to a control. The mice were given the vehicule intra-peritoneally, 2QD.
Composition of the vehicule: 10% DMSO; 10% PEG-400; 30% (EtOH/ Cremophor (1/2)); 50% sterile water.
The second treatment corresponds to the administration of a compound of the invention, compound (01), at a dosage of 18mg/kg 2QD.
The third treatment corresponds to the administration of Taxol at a dosage of 8mg/kg Q3D x5; d1;d4;d7;d10;d13.
The fourth treatment corresponds to the administration of a combination of compound (01) at a dosage of 18mg/kg 2QD and of Taxol at a dosage of 8mg/kg Q3D x5.
The results are depicted in **Fig.2.**
It can be concluded that the compound (01) ((2*E*)-3-(2-furyl)-*N*-({[3-methoxy-4-(2-oxo-2*H*-chromen-3-yl)phenyl] amino}carbonothioyl)acrylamide) inhibits the growth of an aggressive lung tumor xenograft model. It is also observed that the efficacy of compound (01) in reducing the tumour growth is enhanced when combined with another anti-tumour drug, especially when combined with taxol. It has also been observed that the inhibition occurs in a dose-dependent fashion.

**Example 5:** Direct binding of compound (01) on Bcl-xL determined by fluorescence interference Experiments were performed by using a SLM-Aminco 8000C spectrofluorometer with spectral bandwidths of 2 and 4 nm, respectively, for excitation and emission.

0,5 μM purified 6xHis-Bcl-xL were diluted in 1,2 mL of the following buffer pre equilibrated at 25°C (10 mM Tris-HCl, pH= 7.4; 100 mM NaCl; 1 mM β-mercapto ethanol and 10 % glycerol). Fluorescent measurements were performed with increasing concentrations of molecule (01) (from 100 nM to 7,5 μM) in 100 % DMSO
Tryptophan-intrinsic fluorescence of Bcl-xL was measured by scanning emission in the range 310-390 nm upon excitation at 295 nm. The binding of compound (01) was monitored by the increase of fluorescence with increasing concentrations of compound (01).
Correction for innerfilter effect and DMSO dilution were determined using NATA (N-acetyltryptophanamide). All spectra were corrected for the effects of buffer and for dilution. Curve fitting was performed using Grafit (Erithacus, software), with the following quadratic equation for increasing fluorescence:

$$F= Fmin + \{(Fmax - Fmin) [(Et + L + Kd) - ((Et + L + Kd)2 - 4EtL)1/2]\}/2Et$$

Where :

F= relative fluorescence intensity
Fmin= Fluorescence intensity at the start of titration
Fmax= Fluorescence at saturating concentration of compound (01) (Ligand= L)
Et= Total concentration of 6xHis-Bcl-xL
Kd= Apparent dissociation constant of 6xHis-Bcl-xL/ compound (01) complex.

**Example 6:** Pull down assay with small molecules

A peptide aptamer (dubbed BF8) has been selected against the anti-apoptotic protein Bfl1, member of the BCL-2 family. BF8 was shown to interact also with other members of the BCL-2 family, among which Bcl-xL. Two lines of evidence indicate that BF8 binds the hydrophobic groove of anti-apoptotic BCL-2-like proteins. First, the amino-acid sequence of its variable region bears similarities with the sequence of the BH3 domains of pro-apoptotic members of the BCL-2 family (such as Bak or Bax), known to bind the anti-apoptotic proteins via this hydrophobic groove. Second, BF8 no longer binds a mutated variant of Bfl-1 that bears an amino-acid substitution within the said hydrophobic groove.

A Bcl-xL/BF8 *in vitro* interaction assay was developed to examine the capacity of the compounds of the invention to disrupt this interaction by competition with the peptide aptamer. 6xHis-Bcl-xL and GST-BF8 recombinant fusion proteins were expressed in *E.coli* and purified using affinity chromatography. The GST-BF8 fusion protein (or a GST-control aptamer fusion protein) were coupled to glutathione-sepharose solid phases, and soluble 6xHis-Bcl-xL was added to these phases, in presence of different molecules. 6xHis-Bcl-xL molecules captured by the solid phases were revealed by a Western Blot experiment using an anti-6xHis antibody. Details of the protocol are the following:

1- 25 μL glutathion-Sepharose beads pre-rinsed are mixed with pull down buffer (50mM Tris-HCl, pH=8.0; 100mM NaCl; 1mM DTT et 0,01% Tween20) with 10 μG purified GST-BF8 fusion protein. The mixture is incubated at room temperature for half an hour.
2- Beads are washed by centrifugation and dilution in 1 mL pull down buffer. Beads are then collected by centrifugation and the supernatant is removed.
3- 0,5 μG of purified 6xHis-Bcl-xL fusion protein, pre incubated at 4°C for two hours with molecules (50μM) in pull down buffer supplemented with 5% DMSO, is added. The mixture is incubated for two hours under constant agitation.
4- The beads are then washed with three serial concentration/dilution in 1 mL of pull down buffer.
5- The beads are recovered, supernatant is removed and the beads are resuspend in 1x Laemli buffer for SDS-PAGE (0,25 M Tris-HCl, pH= 6,8; 5 % SDS; 0,025 % bromophenol blue; 0.6 M β-mercaptoethanol and 10% glycerol). The mixture is heated for 10 min at 100°C before SDS-PAGE.
6- Western blot analysis is performed using an anti-His tag antibody

From the result shown in figure 3, it can be concluded that compounds (01) ((2*E*)-3-(2-furyl)-*N*-({[3-methoxy-4-(2-oxo-2*H*-chromen-3-yl)phenyl]amino}carbonothioyl)acrylamide) and (02) (*N*-({[3-methoxy-4-(2-oxo-2*H*-chromen-3-yl)phenyl]amino}carbonothioyl)benzamide) inhibit the *in vitro* interaction between 6xHis-Bcl-xL and GST-BF8.

**Example 7:** Apoptosis

In order to demonstrate that the cytotoxic activity of the compounds of this invention is caused by the induction of apoptosis (as can be expected from the targeting of BCL-2-like proteins), three different assays that specifically detect apoptosis were performed on U937 cells treated with compound (01), over a time-course of 24h. The percentage of cells showing activated Caspase 3 and phosphadityl serine exposure at the outer face of the plasma membrane were determined by flow cytometry, according the enclosed protocols. Both assays enable to detect early markers of apoptosis. The percentage of cells exhibiting a sub-G1 phase DNA content was also determined by flow cytometry. Such DNA content is classically used as a marker of apoptosis. In the experiment shown in Fig.4, the percentage of cells exhibiting a G2/M phase DNA content was also determined. Finally, in this same experiment, the percentage of dead cells was determined by incorporation of propidium iodine.

Details of the protocol are the following:

*Cell culture and compound (01) treatment.*

U937 human histiocytic lymphoma were obtained from European Collection of Cell Cultures (ECACC 85011440) and maintained in suspension in RPMI 1640 medium supplemented with 10% heat decomplemented fetal bovine serum, 100 UI/ml penicillin, 100 μg/ml streptomycin and 2mM glutamax I.

Twelve hours before addition of compound (01), U937 cells were seeded in 12 well plates at density of $5x10^5$cells / 900 μl of medium.

Cells were treated by 100 μl of 5μM compound (01) / 1%DMSO (final concentration 0.5μM / 0.1%DMSO) for various time of drug exposure, ranging from 4 hours to 24 hours or DMSO alone for 10 and 24 hours (Control 1, Control 2 respectively)

Wells corresponding to 4, 6, 8, 10 hours of incubation and Control 1 were analyzed on day 1. Wells corresponding to 12, 14, 16, 20, 24 hours of incubation and Control 2 were analyzed on day 2.

*Annexin-V binding analysis and Propidium Iodide staining (PI)* were performed by flow cytometry with Annexin-V FITC kit (IM3546.; Beckman Coulter, Villepinte) according to manufacturer instructions.

Briefly, 5x10$^5$cells were pelleted by centrifugation 1600 rpm / 4°C, resuspended in 100 μl of ice-cold 1X Annexin-V Binding Buffer containing 1 μl of Annexin V-FITC solution and 5 μl of PI (250 μg/ml). After 15 minutes of incubation on ice in the dark, 400 μl of 1X binding buffer were added and cell preparations analysed by flow cytometry.

*Caspase-3,7 activity analysis* was performed with by flow cytometry with Caspglow™ kit (K-183 / biovision ; Mountain View) according to manufacturer instruction.

Briefly, 5x10$^5$cells were pelleted by centrifugation 1600 rpm / 4°C and resuspended in 300 μl cell culture medium. One microliter of FITC-DEVD-FMK was added into each tube and left for 30 minutes at 37°C in incubator with 5% CO2. Cells were centrifuged twice (3000 rpm / 5 minutes) and washed with 0.5 ml of Wash Buffer before being resuspended in 300 μl of Wash buffer and kept on ice until analysis by flow cytometry.

*Flow cytometry.*

All experiments were carried out on a Cytomics Flow Cytometer 500 (Beckman Coulter) equipped with air-cooled Argon ion laser 488nm. All analysis were performed using CXP software (Beckman Coulter).

For each data point, a minimum of 20.000 cells were analysed.

Caspase-3,7 activity and Annexin-V binding were quantified using the FL-1 channel while PI staining was quantified using the FL-4 channel.

*Cell cycle analysis* was performed as previously described.

From the result shown here and in figure 4, it can be concluded that compound (01) (((2*E*)-3-(2-furyl)-*N*-({[3-methoxy-4-(2-oxo-2*H*-chromen-3-yl)phenyl]amino}carbonothioyl)acrylamide)) exerts a cytostatic effect on U937 cells (as revealed by an early increase of the cell population in the G2/M phase of the cell cycle) and induces apoptosis (as revealed by the gradual increase of the percentage of cells exhibiting activated caspase 3, membrane exposure of phosphatidylserine, sub-G1 DNA content).

**Example 8:** Proliferation test

Following the protocol described in Example 2C, different compounds according to the invention have been tested for their cytostatic and cytotoxic activities on mammalian cells in culture (specifically U937 cells).

The.G150 (50% of growth inhibitory) has been determined for all the following compounds, i.e. the dose producing 50% of cell growth inhibition.

The results obtained are given in the following table. Where the G150 provided is in the form of a range, it means that multiple experiments, whose results fall within the range indicated, were performed. Otherwise, results of individual experiments are provided.

| Compound | Structure | U937 G150 (μM) |
|---|---|---|
| Compound 01 | 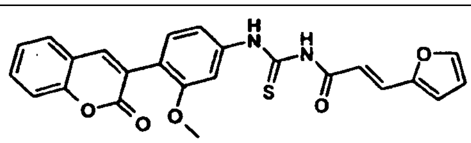 | 0.059 - 0.2 |
| Compound 14 | 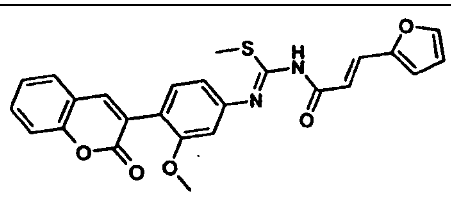 | 0.22 |
| Compound 24 | 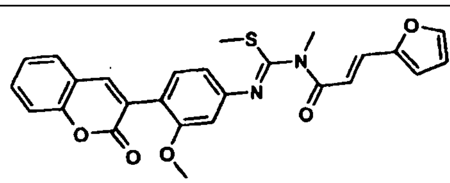 | 0.033 |

(continued)

| Compound | Structure | U937 GI50 (μM) |
|---|---|---|
| Compound 02 | 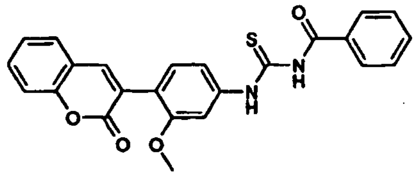 | 0.6 - 0.86 |
| Compound 05 | 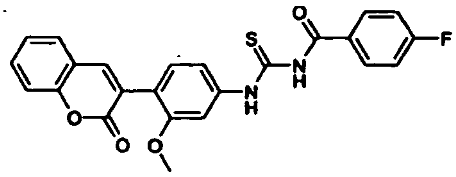 | 0.92, 1.5 |
| Compound 25 | 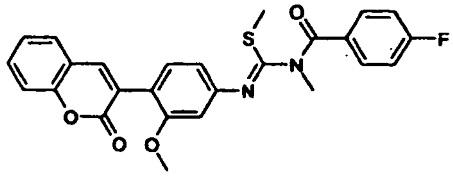 | 0.41 |
| Compound 07 | 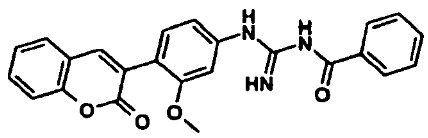 | 0.63, 0.8 |
| Compound 12 | 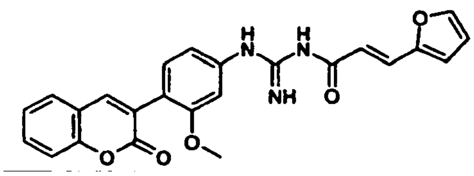 | 1.6-2.1 |
| Compound 19 | 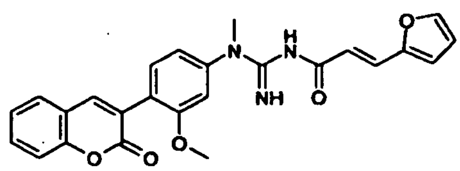 | 9.2 |
| Compound 03 | 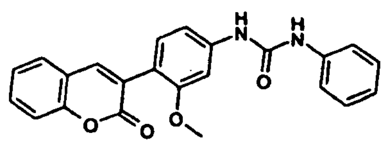 | 0.61 |
| Compound 06 | 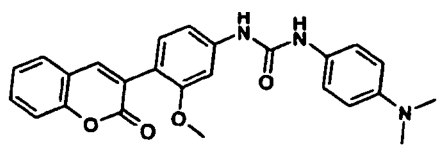 | 0.32, 0.58 |
| Compound 18 | 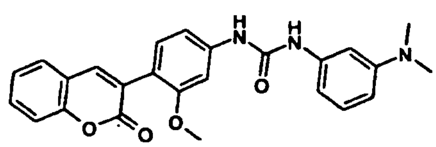 | 0.15 |

(continued)

| Compound | Structure | U937 G150 (μM) |
|---|---|---|
| Compound 08 | 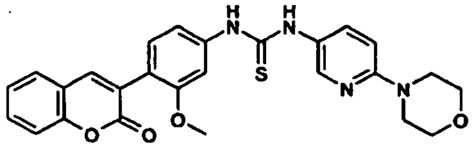 | 1.2 |
| Compound 20 | 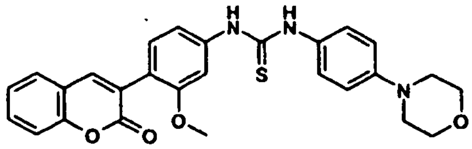 | 1.1 |
| Compound 15 | 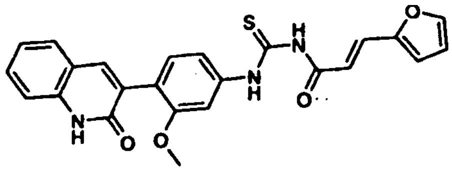 | 0.063, 0.21 |
| Compound 17 | 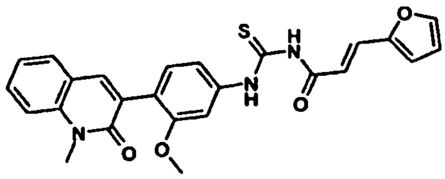 | 0.045 |
| Compound 21 | 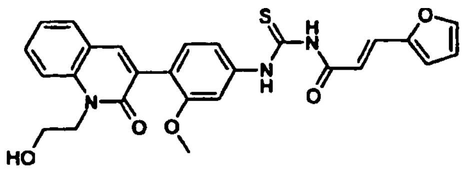 | = 0.09 |
| Compound 22 | 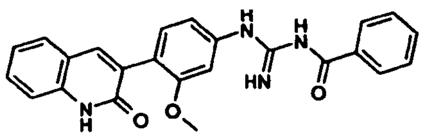 | 1 |
| Compound 09 | 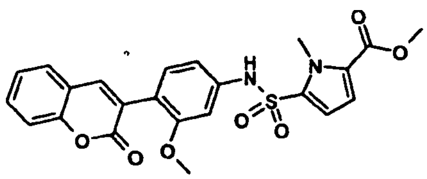 | 0.5 - 0.6 |
| Compound 13 | 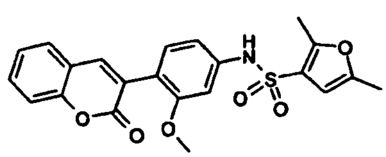 | 0.1 |
| Compound 23 | 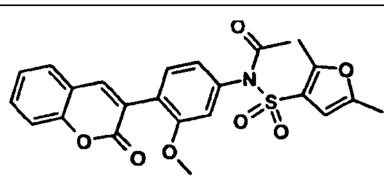 | = 0.5 |

**BIBLIOGRAPHY:**

Contractor R, Konopleva M, Samudio I, et al. Inhibition of Bcl-2 signaling by small molecule BH3 inhibitor GX15-070 as a novel therapeutic strategy in AML. Blood 2005;106:942a, abstract # 3372.

Galan PP, Roue G, Billamor N, et al. The small molecule pan=Bcl-2 inhibitor GX15-070 induces apoptosis in vitro in mantle cell lymphoma (MCL) cells and exhibits a synergistic effect in combination with the proteasome inhibitor bortezomib (Velcade®). Blood. 2005;106:429a, abstract #1490.

Mohammad RM, Wang S, Aboukamee, A. Chen B, Wu X, Chen J, Al-Katib A. Preclinical studies of a nonpeptidic small-molecule inhibitor of Bcl-2 and Bcl-X(L) [(-)-gossypol] against diffuse large cell lymphoma. Mol Cancer Ther 2005 Jan;4(1):13-21.

Oltersdorf T. et al. An inhibitor of Bcl-2 family proteins induces regression of solid tumours. Nature. 2005; 435 (7042): 677-81.

Sattler M. et al. Structure of Bcl-xL-Bak peptide complex: recognition between regulators of apoptosis. Sicence. 1997; 275 (5302): 983-986.

## Claims

**1.** Compound with anti-protiferative properties having one of the general formula:

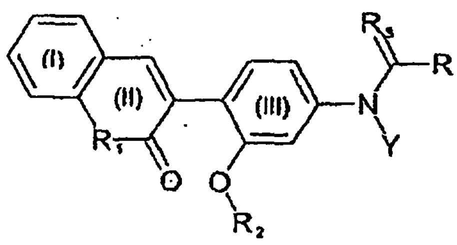

,

formula (a)

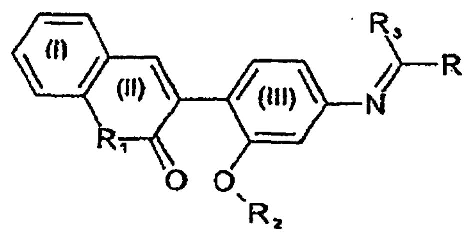

formula (a')

or

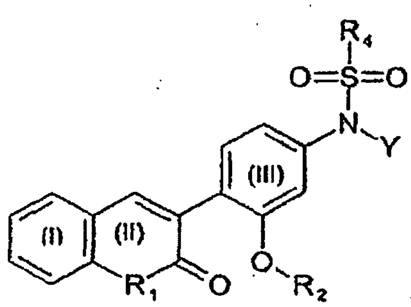

formula (b)

- wherein the cycles (I), (II) and (III) may be optionally substituted on 1, 2, 3 or 4 of the available carbons with a group chosen amongst: Br, Cl, F, I, OH, O-$CH_3$, COOH, and $(CH_2)_m$-$R_9$, wherein $R_9$ represents H, $CH_3$, OH, O-$CH_3$. COOH, $NH_3$, CO-$NH_2$, NH-CO-phenyl, an alkyl, an heteroaryl or an heterocycloalkyl and m=1, 2, 3 or 4; and whereon alkyl is a straight or branched carbon chain that contains from 1 to 12 carbon atoms; heteroaryl is a cyclic group, optionally substituted, having at least one heteroatom selected from O, S or N, said heteroatom interrupt a carbocyclic ring structure containing from 2 to 14 carbon atoms and having a sufficient number of delocalized pl electrons to provide aromatic character: heterocycloalkyl is a saturated, branched or unbranched carbocylic ring containing from 3 to 15 carbon atoms, which carbocyclic ring is interrupted by 1 to 3 hetero croups selected from -O-, -S- or - NR wherein R is - C(O)N(H)$_2$, -$CH_2$C(O)N(H)$_2$, -CHO, -C(O)-O-H, alkyl

containing from 1 to 12 carbo atoms a containing from 6 to 15 carbo atoms, aralkyl, cycloalkyl containing from 3 to 20 carbon atoms, heterocycloalkyl as above defined or heteroaryl as above defined, and being said aralkyl a group made of both aryl and alkyl, groups as above defined;

- wherein Y represents H, $CH_3$ or CO-$CH_3$;
- wherein $R_1$ represents O, $NR_{16}$ with $R_{16}$ being H or an alkyl having from 1 to 4 carbon atoms, or N-$(CH_2)_p$-$R_7$, with $R_7$ representing OH, $COOR_{16}$, CO-$NHR_{16}$, $NHR_{18}$, O-$CH_2R_{16}$, CN, O-CO-$CH_2R_{16}$, CO-heterocycloalkyl being heterocycloalkyl as above defined, a cycloalkyl as above defined, a heterocycloalkyl as above defined, an aryl as above defined or a heteroaryl as above defined, possibly substituted with one or more of the followings radicals: an alkyl having from 1 to 12 carbon atoms, an aryl as above defined, a heteroaryl as above defined, a cycloalkyl as above defined a heterocycloalkyl as above defined, F, Cl, $NH_2$, C≡N, OH, CO-$NH_2$, O-$CH_3$, CO-OH, CO-$OCH_3$ or O-CO-$CH_3$, and p=0.1, 2, 3, 4, 5 or 6:
- wherein $R_2$ represents H or an alkyl with less than 4 carbon atoms,
- wherein $R_3$ represents S. O or NH In formula (a), or S-$(CH_2)_q$-$R_{10}$ or NH-$(CH_2)_4$-$R_{10}$, in formula (a'), wherein $R_{10}$ has the same meaning as $R_9$ and wherein q= 0, 1, 2, 3 or 4;
- wherein $R_4$ represents an alkyl containing from 1 to 12 carbon atoms, aryl as above defined, arylalkyl as above defined, heteroaryl as above defined, heteroarylalkyl, cycloalkyl as above defined, heterocycloalkyl as above defined, or CH=CH-$R_{11}$, wherein $R_{11}$ has the same meaning as $R_4$, and the molecular weight of $R_4$ is less than 500 Da,
- and wherein,

if $R_3$ represents O, then R is N*-$R_5$, or O-$R_5$, wherein $R_6$ has the same meaning as $R_4$ and N* represents NH or N($CH_3$); if $R_3$ represents NH, S-$(CH_2)_qR_{10}$ or NH-$(CH_2)_q$-$R_{10}$, then R is $R_6$, or N*-CO-$R_6$, wherein $R_6$ has the same meaning as $R_4$ and N* represents NH or N($CH_3$); and
if $R_3$ represents S, then R is N*-$(CH_2)_n$-$R_{12}$, wherein $R_{12}$ has the same meaning as $R_7$, n=0, 1, 2, 3 or 4 and N* represents NH or N($CH_3$), or
R is N*-CO-$R_6$, with -$R_8$ representing a linear or branched alkyl having from 1 to 12 carbon atoms, with the exception of tert-butyl, or -$R_6$ represents -$(CH_2)_a$-$C_4H_3O$, with s=1, 2, 3 or 4, or $R_8$ represents a phenyl radical mono-substituted in the para position, with an alkyl, -F, -Cl, -$NH_2$, -C≡N, -OH, -CO-$NH_2$, -O-$CH_3$, -CO-OH, -CO-$OCH_3$ or -O-CO-$CH_3$ and N* represents NH or N($CH_3$), or • R is N*-CO-$R_{17}$, with -$R_{17}$ representing an alkyl having from 1 to 12 carbon atoms, aryl as above defined, arylalkyl as above defined, heteroaryl as above defined, heteroarylalkyl, cycloalkyl as above defined, heterocycloalkyl as above defined, or CH=CH-$R_{18}$, wherein $R_{18}$ has the same meaning as $R_{17}$, and the molecular weight of $R_{17}$ is less than 500 Da, N* represents NH or N($CH_3$) and $R_1$ is not O,
or base or acid addition salt of said compound, or a geometric isomer, an enantiomer or a diastereoisomer thereof.

2. A compound according to claim 1. wherein $R_1$ is an oxygen atom.

3. A compound according to claim 1, wherein $R_2$ is a methyl.

4. A compound according to claim 1, wherein at least one of cycles (I) and (II) is substitute with F or with $(CH_2)_m$-$R_9$, with m=1 or 2 and [$R_9$] is

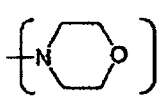 or 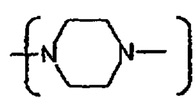.

5. A compound according to claim 1, wherein in formula (a) $R_1$ is O, -$R_2$ is -$CH_3$, $R_3$ is O, Y is H, R is NH-$R_5$ and $R_6$ is a phenyl or $R_5$ is a benzyl or -$R_5$ is -$C_6H_4$-N$(CH_3)_2$.

6. A compound according to claim 1, wherein in formula (a) $R_1$ is O or NH. -$R_2$ is -$CH_3$, $R_3$ is NH, Y is H and [R] is

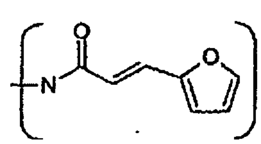,

the furan moiety being optionally replaced by another heterocycloalkyl, <u>selected from</u> thiophene, pyrrole, thiazole,

pyrrazole or oxazole moiety, said moiety being optionally substituted with an alkyl having from 1 to 4 carbon atoms, $NH_3$, O, OH, $CO-NH_2$, $O-CH_3$, CO-OH, $CO-OCH_3$ or $O-CO-CH_3$.

**7.** A compound according to claim 1, wherein in formula (b) $R_1$ is O, $-R_2$ is $-CH_3$, Y is H and ǂ$R_4$] is

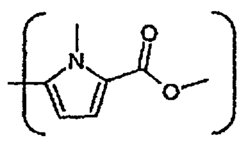
or
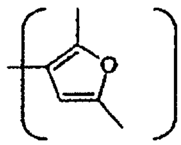
.

**8.** A compound according to claim 1, wherein in formula (a) $R_1$ is O or NH, $-R_2$ is $-CH_3$, $R_3$ is NH, Y is H and -R is $-NH-CO-C_6H_5$.

**9.** A compound according to claim 1, wherein in formula (a) $R_1$ is O, $R_2$ is H or $CH_3$, $R_3$ is S or O, Y is H, R is $NH-(CH_2)_n-R_{12}$, n=0 and ǂ$R_{12}$] is

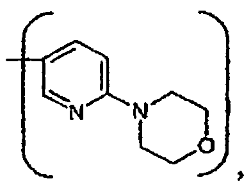
,
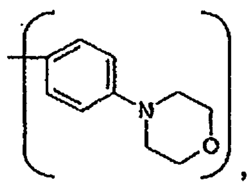
,
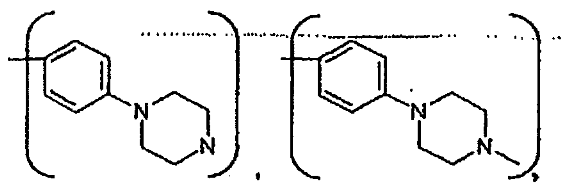
,

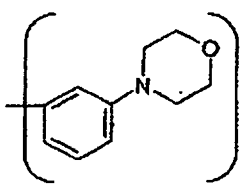
,
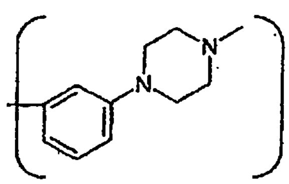
,
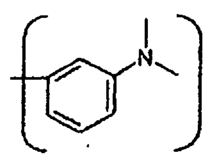
or
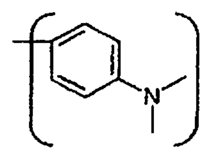
.

**10.** A compound according to claim 1, wherein in formula (a) $R_1$ is O, $-R_2$ is $-CH_3$, $R_3$ is S, Y is H, R is $NH-(CH_2)_n-R_{12}$, n=0 and $R_{12}$ is $C_6H_5$.

**11.** A compound according to claim 1, wherein in formula (a) $R_1$ is O, $-R_2$ is $-CH_3$, $R_3$ is S, Y is H, R is $NH-CO-R_8$ and $-R_8$ is $-CH_2-CH_2-C_4H_3O$ or
ǂ$R_6$] is

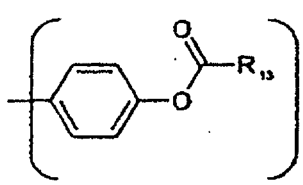

wherein $R_{13}$ is an alkyl in $C_1$ to $C_8$.

**12.** A compound according to claim 1, wherein in formula (a) $R_1$ is O, $-R_2$ is $-CH_3$, $R_3$ is S, R is $NH-(CH_2)_n-R_7$, n=1 and $-R_7$ is $-C_6H_5$.

**13.** A compound according to claim 1, wherein in formula (a') $R_1$ is O, $-R_2$ is $-CH_3$, $R_3$ is $S-CH_3$ and ǂR] is

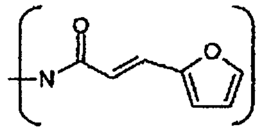 or 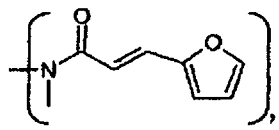,

the furan moiety being optionally replaced by another heterocycloalkyl, selected from thiophene, pyrrole, thiazole, pyrrazole or oxazole moiety, said moiety being optionally substituted with an alkyl having from 1 to 4 carbon atoms, $NH_3$, O, OH; $CO\text{-}NH_2$, $O\text{-}CH_3$, CO-OH, $CO\text{-}OCH_3$ or $O\text{-}CO\text{-}CH_3$.

**14.** A compound according to claim 1, having one of the following formula:

Compound 03: 1-[3-methoxy-4-(2-oxo-2H-chromen-3-yl)phenyl]-3-phenylurea,
Compound 04: 1-[3-methoxy-4-(2-oxo-2H-chromen-3-yl)phenyl]-3-phenylthlourea,
Compound 05: 4-fluoro-*N*-({[3-methoxy-4-(2-oxo-2*H*-chromen-3-yl)phenyl]amino}carbonothioyl)benzamide,
Compound 06: 1-[4-(dimethylamino)phenyl]-3-[3-methoxy-4-(2-oxo-2 *H*-chromen-3-yl)phenyl]urea,
Compound 07: *N*-(imino{[3-methoxy-4-(2-oxo-2*H*-chromen-3-yl)phanyl]amino}methyl)benzamide,
Compound 08: 1-[3-methoxy-4-(2-oxo-2*H*-chromen-3-yl)phenyl]-3-(6-morpholin-4-ylpyridin-3-yl)thiourea,
Compound 09: methyl 5-({[3-methoxy-4-(2-oxo-2*H*-chromen-3-yl)phenyl]amino}sulfonyl)-1-methyl-1 *H*-pyrrole-2-carboxylate,
Compound 10: methyl *N*-[(2*E*)-3-(2-furyl)prop-2-enoyl]-*N*'-[3-methoxy4-(2-oxo-2*H*-chromen-3-yl)phenyl]-N-methylimidothiocarbamate,
Compound 12: (2*E*)-3-(2-furyl)-*N*-(imino{[3-methoxy-4-(2-oxo-2*H*-chromen-3-yl)phenyl]amino}methyl)acrylamide,
Compound 13: *N*-[3-methoxy-4-(2-oxo-2*H*-chromen-3-yl)phenyl]-2,5-dimethylfuran-3-sulfonamide, Compound 14: methyl *N*-[(2*E*)-3-(2-furyl)prop-2-enoyl]-*N*-[3-methoxy-4-(2-oxo-2*H*-chromen-3-yl)phenyl]imidothiocarbamate,
Compound 15: (2*E*)-3-(2-furyl)-*N*-({[3-methoxy-4-(2-oxo-1,2-dihydroquinolin-3-yl)phenyl]amino}carbonothioyl) acrylamide,
Compound 16: methyl *N*-(4-fluorobenzoyl)-*N*'-[3-methoxy-4-(2-oxo-2*H*-chromen-3-yl)phenyl]-*N*-methylimidothiocarbamate,
Compound 17: (2*E*)-3-(2-furyl)-*N*-({[3-methoxy-4-(1-methyl-2-oxo-1,2-dihydroquinolin-3-yl)phenyl]amino}carbonothioyl)acrylamide,
Compound 18: 1-[3-(dimethylamino)phenyl]-3-[3-methoxy-4-(2-oxo-2 *H*-chromen-3-yl)phenyl]urea.
Compound 19: N'-((E)-3-furan-2-yl-acryloyl)-N-[3-methoxy-4-(2-oxo-2H-chromen-3-yl)-phenyl]-N-methyl-guanidine,
Compound 20: 1-[3-methoxy-4-(2-oxo-2H-chromen-3-yl)-phenyl]-3-(4-morpholin-4-yl-phenyl)-thlourea.
Compound 21: 1-((E)-3-furan-2-yl-acryloyl)-3-{4-[1-(2-hydroxy-ethyl)-2-oxo-1,2-dihydro-quinolin-3-yl]-3-methoxy-phenyl}-thiourea,
Compound 22: N-Benzoyl-N'-[3-methoxy-4-(2-oxo-1,2-dihydro-quinolin-3-yl)-phenyl]-guanidine,
Compound 23: 2,5-dimethyl-furan-3-sulfonic acid acetyl-[3-methoxy-4-(2-oxo-2H-chromen-3-yl)-phenyl]-amide,
Compound 24: 1-((E)-3-furan-2-yl-acryloyl)-3-[3-methoxy-4-(2-oxo-2H-chromen-3-yl)-phenyl]-1,2-dimethyl-isothiourea, and
Compound 25: 1-(4-fluoro-benzoyl)-3-[3-mathoxy-4-(2-oxo-2H-chromen-3-yl)-phenyl]-1,2-dimethyl-isothipurea.

**15.** A compound according to any one of claims 1 to 14, having cytotoxic properties on tumoral cell.

**16.** A compound according to any one of claims 1 to 11, having cytostatic properties on tumoral cell.

**17.** A compound according to claim 15 or 16, wherein said tumoral cells are lung, prostate, breast, colon, melanoma, lymphoma or myeloid tumor cells.

**18.** A compound according to any one of claims 1 to 14 which is soluble in water at molarity above $50.10^{-6}$ $mol.L^{-1}$.

**19.** A compound according to any one of claims 1 to 18 which binds Bcl-xL protein with a Kd less than 1$\mu$M.

**20.** A composition comprising a compound according to any one of claims 1 to 19 in association with at least one other anti-tumor agent.

**21.** A composition according to claim 20, wherein said anti-tumor agent is taxol, etoposide, doxorubicin, cis-platin, retinoic acid, tamoxifen, vinblastin, vincristin, cyclophosphamide, topotecan, irinotecan, gemcitabine or rapamycin.

**22.** A product containing a compound according to any one of claims 1 to 19 and a further anti-tumor agent as a combined preparation for simultaneous, separate or sequential use in therapy.

**23.** A pharmaceutical composition comprising a compound according to any one of claims 1 to 19 and a pharmaceutically acceptable carrier.

**24.** A pharmaceutical composition according to claim 23, which is to be administered via intralesional, intraperitoneal, intramuscular or intravenous injection; via infusion; liposome-mediated delivery; topical, nasal, oral, anal, subcutaneous, vaginal, sublingual, uretheral, transdermal, intrathecal, ocular or otic delivery.

**25.** Use of a compound according to any one of claims 1 to 19, for the manufacture of a medicament to be administered to a subject for treating cancer.

**26.** Use according to claim 26, wherein said medicament is to be administered via intralesional; intraperitoneal, intramuscular or intravenous injection: via infusion; liposome-mediated delivery; topical, nasal, oral, anal, subcutaneous, vaginal, sublingual, uretheral, transdermal, intrathecal, ocular or otic delivery.

**27.** Use of a composition according to claim 20 or 21, for the manufacture of a medicament to be administered to a subject for treating cancer.

**28.** Use of a compounds according to any one of claims 1 to 19 for the manufacture of a medicament to be administered simultaneously, separately or sequentially with at least one other anti-tumor agent, to a subject for treating cancer.

**29.** Process for inhibiting cell proliferation *in vitro* comprising contacting a cell *in vitro,* with a compound according to any one of claims 1 to 19.

**Patentansprüche**

**1.** Verbindung mit anti-proliferativen Eigenschaften, welche eine der allgemeinen Formeln aufweist:

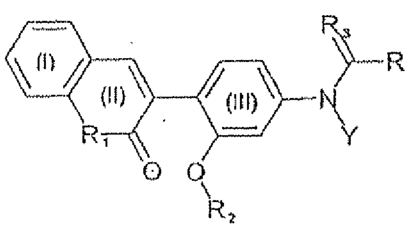

,

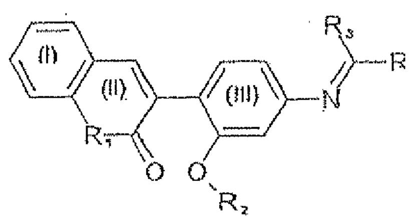

Formel (a)    Formel (a')

oder

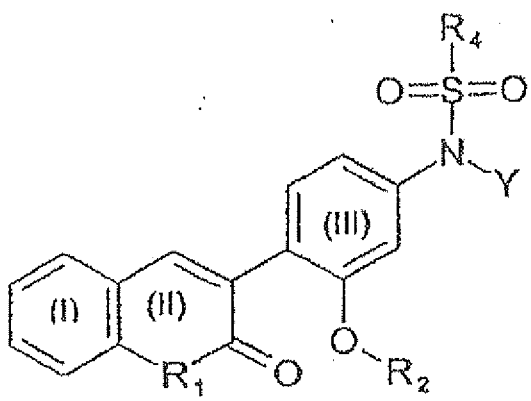

Formel (b)

- worin die Cyclen (I), (II) und (III) gegebenenfalls substituiert sein können an 1, 2, 3 oder 4 der verfügbaren Kohlenstoffatome mit einer Gruppe, ausgewählt aus: Br, Cl, F, I, OH, O-$CH_3$, COOH und $(CH_2)_m$-$R_9$, worin $R_9$ darstellt H, $CH_3$, OH, O-$CH_3$, COOH, $NH_3$, CO-$NH_2$, NH-CO-Phenyl, ein Alkyl, ein Heteroaryl oder ein Heterocycloalkyl und m gleich 1, 2, 3 oder 4 ist; und worin Alkyl eine geradkettige oder verzweigte Kohlenstoffkette ist, die 1 bis 12 Kohlenstoffatome enthält; Heteroaryl eine gegebenenfalls substituierte cyclische Gruppe mit mindestens einem Heteroatom, ausgewählt aus O, N oder N ist, wobei das Heteroatom eine carbocyclische Ringstruktur, enthaltend 2 bis 14 Kohlenstoffatome und mit einer ausreichenden Anzahl an delokalisierten pi-Elektroden, um einen aromatischen Charakter zu erzeugen, unterbricht; Heterocycloalkyl ein gesättigter, geradliniger oder verzweigter carbocyclischer Ring ist, welcher 3 bis 15 Kohlenstoffatome umfasst, wobei der carbocyclische Ring durch 1 bis 3 Heterogruppen unterbrochen ist, wobei die Heterogruppen ausgewählt sind aus -O-, -S- oder -NR-, worin R gleich -C(O)N$(H)_2$, -$CH_2$C(O)N$(H)_2$, -CHO, -C(O)-O-H, Alkyl, umfassend 1 bis 12 Kohlenstoffatome, Aryl, umfassend 6 bis 15 Kohlenstoffatome, Aralkyl, Cycloalkyl, umfassend 3 bis 20 Kohlenstoffatome, wie vorstehend definiertes Heterocycloalkyl oder wie vorstehend definiertes Heteroaryl ist und wobei die Aralkylgruppe eine Gruppe ist, die sowohl aus den vorstehend definierten Aryl- als auch Alkylgruppen besteht;
- worin Y darstellt H, $CH_3$ oder CO-$CH_3$;
- worin $R_1$ gleich O, $NR_{16}$ ist mit $R_{16}$ gleich Wasserstoff oder Alkyl mit 1 bis 4 Kohlenstoffatomen oder N-$(CH_2)_p$-$R_7$ mit $R_7$ gleich OH, $COOR_{16}$, CO-$NHR_{16}$, $NHR_{16}$, O-$CH_2R_{16}$, CN, O-CO-$CH_2R_{16}$, CO-Heterocycloalkyl, wobei Heterocycloalkyl wie vorstehend definiert ist, ein Cycloalkyl wie vorstehend definiert, ein Heterocycloalkyl wie vorstehend definiert, ein Aryl wie vorstehend definiert oder ein Heteroaryl wie vorstehend definiert, weiche gegebenenfalls mit einem oder mehreren der folgenden Reste substituiert sind: einem Alkyl mit 1 bis 12 Kohlenstoffatomen, einem Aryl wie vorstehend definiert, einem Heteroaryl wie vorstehend definiert, einem Cycloalkyl wie vorstehend definiert, einem Heterocycloalkyl wie vorstehend definiert, F, Cl, $NH_2$, C = N, OH, CO-$NH_2$, O-$CH_3$, CO-OH, CO-$OCH_3$ oder O-CO-$CH_3$ und p gleich 0, 1, 2, 3, 4, 5 oder 6 ist;
- worin $R_2$ gleich Wasserstoff oder ein Alkyl mit weniger als 4 Kohlenstoffatomen ist,
- worin $R_3$ gleich S, O oder NH in der Formel (a) oder S-$(CH_2)_q$-$R_{10}$ oder NH-$(CH_2)_q$-$R_{10}$ in der Formel (a') darstellt, worin $R_{10}$ dieselbe Bedeutung wir $R_9$ aufweist und worin q gleich 0, 1, 2, 3 oder 4 ist;
- worin $R_4$ gleich Alkyl mit 1 bis 12 Kohlenstoffatomen, Aryl wie vorstehend definiert, Aralkyl wie vorstehend definiert, Heteroaryl wie vorstehend definiert, Heteroarylalkyl, Cycloalkyl wie vorstehend definiert, Heterocycloalkyl wie vorstehend definiert oder CH = CH-$R_{11}$ ist, worin $R_{11}$ dieselbe Bedeutung wie $R_4$ aufweist und das Molekulargewicht von $R_4$ geringer als 500 Da ist,
- und worin

wenn $R_3$ gleich O ist, dann R gleich N*-$R_5$ oder O-$R_5$ ist, worin $R_5$ dieselbe Bedeutung wie $R_4$ aufweist und N* gleich NH oder N$(CH_3)$ ist;
wenn $R_3$ gleich NH, S-$(CH_2)_q$-$R_{10}$ oder NH-$(CH_2)_q$-$R_{10}$ ist, dann R gleich $R_6$ oder N*-CO-$R_6$ ist, worin $R_6$ dieselbe Bedeutung wie $R_4$ aufweist und N* gleich NH oder N$(CH_3)$ ist; und
wenn $R_3$ gleich S darstellt, dann ist

• R gleich N*-$(CH_2)_n$-$R_{12}$ ist, worin $R_{12}$ dieselbe Bedeutung wie $R_7$ ufweist, n gleich 0, 1, 2, 3 oder 4 ist und N*

gleich NH oder $N(CH_3)$ ist oder

• R gleich N*-CO-$R_8$ ist, wobei -$R_8$ einem linearen oder verzweigten Alkylrest mit 1 bis 12 Kohlenstoffatomen entspricht mit der Ausnahme von tert.-Butyl oder -$R_8$ gleich $-(CH_2)_s-C_4H_3O$ mit s gleich 1, 2, 3 oder 4 ist oder $R_8$ einen Phenylrest darstellt, welcher in der para-Position monosubstituiert ist mit Alkyl, -F, -Cl, $-NH_2$, -C ≡ N, -OH, $-CO-NH_2$, $-O-CH_3$, -CO-OH, $-CO-OCH_3$ oder $-O-CO-CH_3$ und N* gleich NH oder $N(CH_3)$ ist, oder

• R gleich N*-CO-$R_{17}$ ist mit -$R_{17}$ gleich Alkyl mit 1 bis 12 Kohlenstoffatomen, Aryl wie vorstehend definiert, Aralkyl wie vorstehend definiert, Heteroaryl wie vorstehend definiert, Heteroarylalkyl, Cycloalkyl wie vorstehend definiert, Heterocycloalkyl wie vorstehend definiert oder CH = CH-$R_{18}$, worin $R_{18}$ dieselbe Bedeutung wie $R_{17}$ aufweist und das Molekulargewicht von $R_{17}$ geringer als 500 Da ist, N* gleich NH oder $N(CH_3)$ darstellt und $R_1$ ungleich O ist,

oder ein basisches oder saures Additionssalz dieser Verbindung oder ein geometrisches Isomer, ein Enantiomer oder ein Diastereomer davon.

**2.** Eine Verbindung nach Anspruch 1, worin $R_1$ ein Sauerstoffatom darstellt.

**3.** Eine Verbindung nach Anspruch 1, worin $R_2$ einem Methyl entspricht.

**4.** Eine Verbindung nach Anspruch 1, worin mindestens eines der Cyclen (I) und (II) substituiert ist mit F oder mit $(CH_2)_m-R_9$ mit m = 1 oder 2 und (-$R_9$) gleich

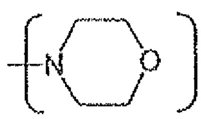

oder

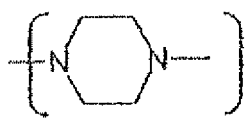

ist.

**5.** Eine Verbindung nach Anspruch 1, worin in Formel (a) $R_1$ gleich O ist, -$R_2$ gleich $-CH_3$ ist, $R_3$ gleich O ist, Y gleich H ist, R gleich NH-$R_5$ ist und $R_5$ gleich einem Phenyl ist oder $R_5$ gleich einem Benzyl ist oder -$R_5$ gleich $-C_6H_4-N(CH_3)_2$ ist,

**6.** Eine Verbindung nach Anspruch 1, worin in der Formel (a) $R_1$ gleich O oder NH ist, -$R_2$ gleich $-CH_3$ ist, $R_3$ gleich NH ist, Y gleich H ist und -(R) gleich

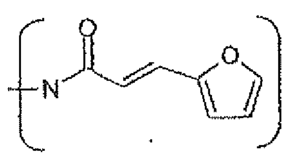

ist, wobei der Furanring gegebenenfalls durch einen anderen Heterocycloalkylrest ausgetauscht sein kann, welcher ausgewählt ist aus einem Thiophenring, einem Pyrrolring, einem Thiazolring, einem Pyrrazolring oder einem Oxazolring, wobei der Rest gegebenenfalls substituiert sein kann mit Alkyl mit 1 bis 4 Kohlenstoffatomen, $NH_3$, O, OH, $CO-NH_2$, $O-CH_3$, -CO-OH, $CO-OCH_3$ oder $O-CO-CH_3$.

**7.** Eine Verbindung nach Anspruch 1, worin in Formel (b) $R_1$ gleich O ist, -$R_2$ gleich $-CH_3$ ist, Y gleich H ist und -($R_4$) gleich

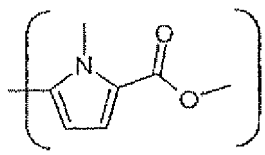

oder

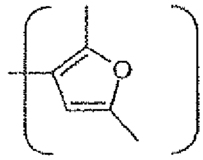

ist,

**8.** Eine Verbindung nach Anspruch 1, worin in der Formel (a) $R_1$ gleich O oder NH ist, $-R_2$ gleich $-CH_3$ ist, $R_3$ gleich NH ist, Y gleich H ist und -R gleich $-NH-CO-C_6H_5$ ist,

**9.** Eine Verbindung nach Anspruch 1, worin in der Formel (a) $R_1$ gleich O ist, $R_2$ gleich H oder $CH_3$ ist, $R_3$ gleich S oder O ist, Y gleich H ist, R gleich $NH-(CH_2)_n-R_{12}$ ist, n gleich 0 ist und $-(R_{12})$ gleich

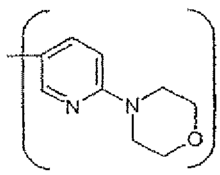
,
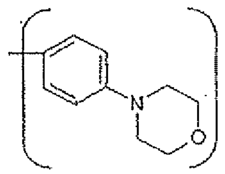
,
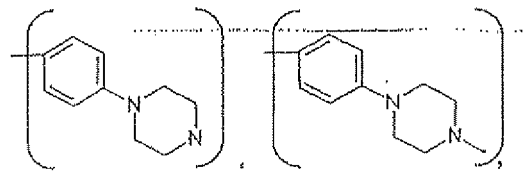
,

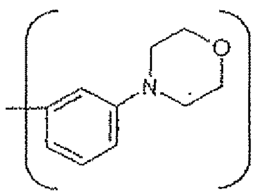
,
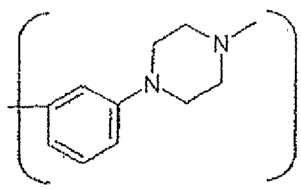
,
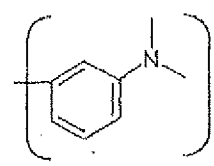
oder
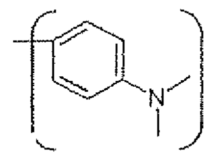
.

ist.

**10.** Eine Verbindung nach Anspruch 1, worin in Formel (a) $R_1$ gleich O ist, $-R_2$ gleich $-CH_3$ ist, $R_3$ gleich S ist, Y gleich H ist, R gleich $NH-(CH_2)_n-R_{12}$ ist, n gleich 0 ist und $R_{12}$ gleich $C_6H_5$ ist.

**11.** Eine Verbindung nach Anspruch 1, worin in Formel (a) $R_1$ gleich O ist, $-R_2$ gleich $-CH_3$ ist, $R_3$ gleich S ist, Y gleich H ist, R gleich $NH-CO-R_8$ ist und $-R_8$ gleich $-CH_2-CH_2-C_4H_3O$ ist oder $(-R_8)$ gleich

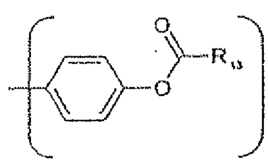

ist, worin $R_{13}$ ein $(C_1-C_6)$-Alkyl ist,

**12.** Eine Verbindung nach Anspruch 1, worin in Formel (a) $R_1$ gleich 0 ist, $-R_2$ gleich $-CH_3$ ist, $R_2$ gleich S ist, R gleich $NH-(CH_2)_n-R_7$ ist, n gleich 1 ist und $-R_7$ gleich $-C_6H_5$ ist.

**13.** Eine Verbindung nach Anspruch 1 , worin in der Formel (a') $R_1$ gleich O ist, $-R_2$ gleich $-CH_3$ ist, $R_3$ gleich $S-CH_3$ ist

und -(R) gleich

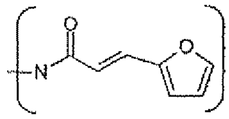

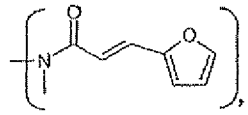

oder

ist, wobei der Furanrest gegebenenfalls ausgetauscht sein kann durch einen anderen Heterocycloalkylrest, ausgewählt aus einem Thiophenring, einem Pyrrolring, einem Thiazolring, einem Pyrrazolring oder einem Oxazolring, wobei der Ring gegebenenfalls substituiert sein kann mit einem Alkyl mit 1 bis 4 Kohlenstoffatomen, $NH_3$, O, OH, $CO\text{-}NH_2$, $O\text{-}CH_3$, CO-OH, $CO\text{-}OCH_3$ oder $O\text{-}CO\text{-}CH_3$.

**14.** Eine Verbindung nach Anspruch 1, welche eine der folgenden Formeln aufweist:

Verbindung 03: 1-[3-Methoxy-4-(2-oxo-2H-chromen-3-yl)phenyl]-3-phenylharnstoff,
Verbindung 04: 1-[3-Methoxy-4-(2-oxo-2H-chromen-3-yl)phenyl]-3-phenylthioharnsfoff,
Verbindung 05: 4-Fluoro-N-({[3-methoxy-4-(2-oxo-2H-chromen-3-yl)phenyl]amino}carbonothioyl)benzamid,
Verbindung 06: 1-[4-(Dimethylamino)phenyl]-3-[3-methoxy-4-(2-oxo-2H-chromen-3-yl) phenyl] harnstoff,
Verbindung 07: N-(Imino{[3-methoxy-4-(2-oxo-2H-chromen-3-yl)phenyl]amino}methyl)benzamid,
Verbindung 08: 1-[3-Methoxy-4-(2-oxo-2H-chromen-3-yl)phenyl]-3-(6-morpholin-4-ylpyridin-3-yl)thioharnstoff,
Verbindung 09: Methyl-5-({[3-methoxy-4-(2-oxo-2H-chromen-3-yl)phenyl]amino}sulfonyl)-1-methyl-1-H-pyrrol-2-carboxylat,
Verbindung 10: Methyl-N-[(2E)-3-(2-furyl)prop-2-enoyl]-N'-[3-methoxy-4-(2-oxo-2H-chromen-3-yl) phenyl]-N-methylimidothiocarbamat,
Verbindung 12: (2E)-3-(2-Furyl)-N-(imino{[3-methoxy-4-(2-oxo-2H-chromen-3-yl) phenyl] amino}methyl) acrylamid,
Verbindung 13: N-[3-Methoxy-4-(2-oxo-2H-chromen-3-yl)phenyl]-2,5-dimethylfuran-3-sulfonamid,
Verbindung 14: Methyl-N-[(2E)-3-(2-furyl)prop-2-enoyl]-N'-[3-methoxy-4-(2-oxo-2H-chromen-3-yl) phenyl] imidothiocarbamat,
Verbindung 15: (2E)-3-(2-Furyl)-N-({[3-methoxy-4-(2-oxo-1,2-dihydrochinolin-3-yl)phenyl]amino}carbonothioyl) acrylamid,
Verbindung 16: Methyl-N-(4-fluorobenzoyl)-N'-[3-methoxy-4-(2-oxo-2H-chromen-3-yl)phenyl]-N-methylimidothiocarbamat,
Verbindung 17: (2E)-3-(2-Furyl)-N-({[3-methoxy-4-(1-methyl-2-oxo-1,2-dihydrochinolin-3-yl)phenyl]amino}carbonothioyl)acrylamid,
Verbindung 18: 1-[3-(Dimethylamino)phenyl]-3-[3-methoxy-4-(2-oxo-2H-chromen-3-yl]phenyl]harnstoff,
Verbindung 19: N'-((E)-3-Fluran-2-yl-acryloyl)-N-[3-methoxy-4-(2-oxo-2H-chromen-3-yl) phenyf]-N-rnethylguanidin,
Verbindung 20; 1-[3-Methoxy-4-(2-oxo-2H-chromen-3-yl)phenyl]-3-(4-morpholin-4-yl-phenyl)-thioharnstoff,
Verbindung 21 ; 1 -((E)-3-Furan-2-yl-acryloyl)-3-[4-[1-(2-hydroxy-ethyl)-2-oxo-1,2-dihydio-chinolin-3-yl]-3-methoxy-phenyl]-thioharnstoff,
Verbindung 22: N-Benzoyl-N'-[3-methoxy-4-(2-oxo-1,2-dihydro-chinolin-3-yl)-phenyl]guanidin,
Verbindung 23: 2,5-Dimethyl-furan-3-sulfonsäureacetyl-[3-methoxy-4-(2-oxo-2H-chromen-3-yl)-phenyl]-amid,
Verbindung 24: 1 -((E)-3-Furan-2-yl-acryloyl)-3-[3--methoxy-4-(2-oxo-2H-chromen-3-yl)-phenyl]-1,2-dimethylisothioharnstoff und
Verbindung 25: 1-(4-Fluoro-benzoyl)-3-[3-methoxy-4-(2-oxo-2H-chromen-3-yl)-phenyl]-1,2-dimethyl-isothioharnstoff.

**15.** Eine Verbindung nach einem der Ansprüche 1 bis 14 mit cytotoxischen Eigenschaften auf Tumorzellen.

**16.** Eine Verbindung nach einem der Ansprüche 1 bis 14 mit cytostatischen Eigenschaften auf Tumorzellen,

**17.** Eine Verbindung nach Anspruch 15 oder 16, worin die Tumorzellen Lungentumorzellen, Prostatatumorzellen, Brusttumorzellen, Darmtumorzellen, Melanomtumorzelien, Lymphomtumorzellen oder Knochenmarktumorzellen sind,

**18.** Eine Verbindung nach einem der Ansprüche 1 bis 14, welche löslich ist in Wasser mit einer Molarität von oberhalb $15 \cdot 10^{-6}$ $mol/L^{-1}$,

**19.** Eine Verbindung nach einem der Ansprüche 1 bis 18, welche an das Bcl-xL-Protein mit einem Kd von weniger als 1 μM bindet.

**20.** Eine Zusammensetzung, umfassend eine Verbindung nach einem der Ansprüche 1 bis 19 in Kombination mit mindestens einem weiteren Antitumormittel.

**21.** Eine Zusammensetzung nach Anspruch 20, worin das Antitumormittel Taxol, Etoposid, Doxorubicin, cis-Platin, Retinolsäure, Tamoxifen, Vinblastin, Vincristin, Cyclophosphamid, Topotecan, Irinotecan, Gemcitabin oder Rapamycin ist,

**22.** Ein Produkt, umfassend eine Verbindung gemäß einem der Ansprüche 1 bis 19 und ein weiteres Antitumormittel als eine kombinierte Präparation für die gleichzeitige, separate oder aufeinanderfolgende Verwendung in der Therapie,

**23.** Eine pharmazeutische Zusammensetzung, umfassend eine Verbindung gemäß einem der Ansprüche 1 bis 19 und einen pharmazeutisch annehmbaren Träger.

**24.** Eine pharmazeutische Zusammensetzung nach Anspruch 23, welche verabreicht wird über eine intralesionale Injektion, intraperitoneale Injektion, intramuskuläre Injektion oder intravenöse Injektion, über eine Infusion; über eine Liposom-vermittelte Zuführung, topisch, nasal, oral, anal, subkutan, vaginal, sublingual, uretheral, transdermal, intraketal, okkular oder über eine otogene Zuführung.

**25.** Verwendung einer Verbindung mit einem der Ansprüche 1 bis 19 für die Verstellung eines Medikaments zur Verabreichung an einem Subjekt zur Behandlung des Krebs.

**26.** Verwendung nach Anspruch 25, worin das Medikament verabreicht wird über eine intralesionale Injektion, intraperitoneale Injektion, intramuskuläre Injektion oder intravenöse Injektion, über eine Infusion; über eine Liposom-vermittelte Zuführung, topisch, nasal, oral, anal, subkutan, vaginal, sublingual, uretheral, transdermal, intraketal, okkular oder über eine otogene Zuführung,

**27.** Verwendung einer Zusammensetzung nach Anspruch 20 oder 21 für die Herstellung eines Medikaments zur Verabreichung an einem Subjekt zur Behandlung von Krebs,

**28.** Verwendung einer Verbindung gemäß einem der Ansprüche 1 bis 19 für die Verstellung eines Medikaments zur gleichzeitigen, separaten oder sequenziellen Verabreichung mit mindestens einem weiteren Antitumormittel an einem Subjekt für die Behandlung von Krebs.

**29.** Verfahren zur Inhibierung des Zellwachstums in vitro, umfassend das Inkontaktbringen einer Zelle in vitro mit einer Verbindung gemäß einem der Ansprüche 1 bis 19.

## Revendications

**1.** Composé possédant des propriétés anti-prolifératives ayant l'une des formules générales :

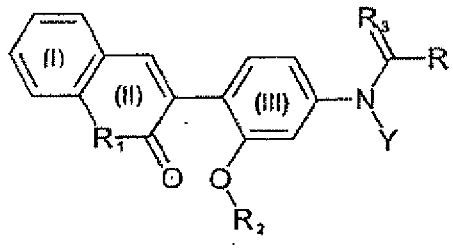

formule (a)

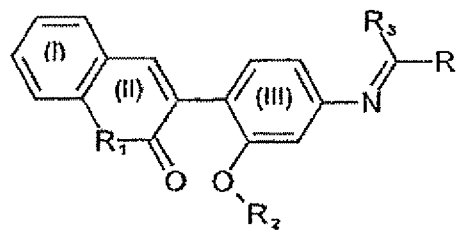

formule (a')

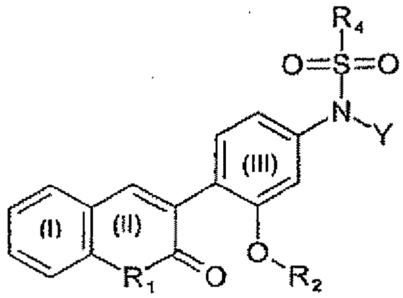

ou formule (b)

dans lesquelles les cycles (I), (II) et (III) peuvent être éventuellement substitués sur 1, 2, 3 ou 4 des atomes de carbone disponibles par un groupe choisi parmi Br, Cl, F, I, OH, $OCH_3$, COOH et $(CH_2)_m$-$R_9$, $R_9$ représentant H, $CH_3$, OH, $OCH_3$, COOH, $NH_3$, $CONH_2$, NH-CO-phényle, un radical alkyle, un radical hétéroaryle ou un radical hétérocycloalkyle et m = 1, 2, 3 ou 4 ; et dans lesquelles un radical allyle est une chaîne carbonée linéaire ou ramifiée contenant de 1 à 12 atomes de carbone ; un radical hétéroaryle est un groupe cyclique, éventuellement substitué, ayant au moins un hétéroatome choisi parmi O, S ou N, ledit hétéroatome interrompant une structure de cycle carbocyclique contenant de 2 à 14 atomes de carbone et ayant un nombre suffisant d'électrons pi délocalisés pour conférer un caractère aromatique ; un radical hétérocycloalkyle est un cycle carbocyclique saturé, ramifié ou non ramifié, contenant de 3 à 15 atomes de carbone, lequel cycle carbocyclique est interrompu par 1 à 3 groupes hétéroatomiques choisis parmi O, S ou NR, R étant $C(O)N(H)_2$, $CH_2C(O)N(H)_2$, CHO, C(O)OH, un radical allyle contenant de 1 à 12 atomes de carbone, un radical aryle contenant de 6 à 15 atomes de carbone, un radical aralkyle, un radical cycloalkyle contenant de 3 à 20 atomes de carbone, un radical hétérocycloalkyle tel que défini ci-dessus ou un radical hétéroaryle tel que défini ci-dessus, et ledit radical aralkyle étant un groupe constitué à la fois de radicaux aryles et alkyles tels que définis ci-dessus ;

dans lesquelles Y représente H, $CH_3$ ou $COCH_3$ ;

dans lesquelles $R_1$ représente O, $NR_{16}$, $R_{16}$ étant H ou un radical alkyle ayant de 1 à 4 atomes de carbone, ou N-$(CH_2)_p$-$R_7$, $R_7$ représentant OH, $COOR_{16}$, $CONHR_{16}$, $NHR_{16}$, $OCH_2R_{16}$, CN, $OCOCH_2R_{16}$, CO-hétérocycloalkyle avec un radical hétérocycloalkyle tel que défini ci-dessus, un radical cycloalkyle tel que défini ci-dessus, un radical hétérocycloalkyle tel que défini ci-dessus, un radical aryle tel que défini ci-dessus ou un radical hétéroaryle tel que défini ci-dessus, éventuellement substitué par un ou plusieurs des radicaux suivants: un radical allyle ayant de 1 à 12 atomes de carbone, un radical aryle tel que défini ci-dessus, un radical hétéroaryle tel que défini ci-dessus, un radical cycloalkyle tel que défini ci-dessus, un radical hétérocycloalkyle tel que défini ci-dessus, F, Cl, $NH_2$, C≡N, OH, $CONH_2$, $OCH_3$, COOH, $COOCH_3$ ou $OCOCH_3$ et p = 0, 1, 2, 3, 4, 5 ou 6 ;

dans lesquelles $R_2$ représente H ou un radical alkyle de moins de 4 atomes de carbone,

dans lesquelles $R_3$ représente S, O ou NH dans la formule (a) ou S-$(CH_2)_q$-$R_{10}$ ou NH-$(CH_2)_q$-$R_{10}$ dans la formule (a'), $R_{10}$ ayant la même signification que $R_9$ et q = 0, 1, 2, 3 ou 4;

dans lesquelles $R_4$ représente un radical alkyle contenant de 1 à 12 atomes de carbone, un radical aryle tel que défini ci-dessus, un radical arylalkyle tel que défini ci-dessus, un radical hétéroaryle tel que défini ci-dessus, un radical hétéroarylalkyle, un radical cycloalkyl, tel que défini ci-dessus, un radical hétérocycloalkyle tel que défini ci-dessus ou CH=CH-$R_{11}$, $R_{11}$ ayant la même signification que $R_4$ et la masse moléculaire de $R_4$ étant inférieure à 500 Da ;

et dans lesquelles,

si $R_3$ représente O, alors R est N*-$R_5$ ou $OR_5$, $R_5$ ayant la même signification que $R_4$ et N* représentant NH ou $N(CH_3)$;

si $R_3$ représente NH, S-$(CH_2)_q$-$R_{10}$ ou NH-$(CH_2)_q$-$R_{10}$, alors R est $R_6$ ou N*-CO-$R_6$, $R_6$ ayant la même signification que $R_4$ et N* représentant NH ou N($CH_3$) ; et
si $R_3$ représente S, alors

- R est N*-$(CH_2)_n$-$R_{12}$; $R_{12}$ ayant la même signification que $R_7$, n = 0, 1, 2, 3 ou 4 et N* représentant NH ou N ($CH_3$), ou
- R est N*-CO-$R_8$, $R_8$ représentant un radical alkyle linéaire ou ramifié ayant de 1 à 12 atomes de carbone, à l'exception du radical tert-butyle, ou $R_8$ représente - $(CH_2)_5$-$C_4H_3O$, avec s = 1, 2, 3 ou 4, ou $R_8$ représentant un radical phényle monosubstitué en position para par un radical alkyle, F, Cl, $NH_2$, C ≡N, OH, $CONH_2$, $OCH_3$, COOH, $COOCH_3$ ou $OCOCH_3$ et N* représentant NH ou N($CH_3$), ou
- R est N*-CO-$R_{17}$, $R_{17}$ représentant un radical alkyle ayant de 1 à 12 atomes de carbone, un radical aryle tel que défini ci-dessus, un radical arylalkyle tel que défini ci-dessus, un radical hétéroaryle tel que défini ci-dessus, un radical hétéroarylalkyle, un radical cycloalkyle tel que défini ci-dessus, un radical hétérocycloalkyle tel que défini ci-dessus, ou CH=CH-$R_{18}$, $R_{18}$ ayant la même signification que $R_{17}$ et la masse moléculaire de $R_{17}$ étant inférieure à 500 Da, et N* représentant NH ou N($CH_3$) et $R_1$ n'étant pas O,

ou un sel d'addition acide ou basique dudit composé ou un isomère géométrique, un énantiomère ou un diastéréoisomère de celui-ci.

**2.** Composé selon la revendication 1, dans lequel $R_1$ est un atome d'oxygène.

**3.** Composé selon la revendication 1, dans lequel $R_2$ est un radical méthyle.

**4.** Composé selon la revendication 1, dans lequel au moins l'un des cycles (I) et (II) est substitué par F ou par $(CH_2)_m$-$R_9$, avec m = 1 ou 2 et -[$R_9$] étant

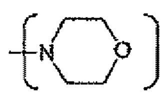
ou
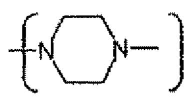
.

**5.** Composé selon la revendication 1, dans lequel dans la formule (a), $R_1$ est O, $R_2$ est $CH_3$, $R_3$ est 0, Y est H, R est NH-$R_5$ et $R_5$ est un radical phényle ou $R_5$ est un radical benzoyle ou $R_5$ est -$C_6H_4$-N$(CH_3)_2$.

**6.** Composé selon la revendication 1, dans lequel dans la formole (a), $R_1$ est O ou NH, $R_2$ est $CH_3$, $R_3$ est NH, Y est H et -[R] est

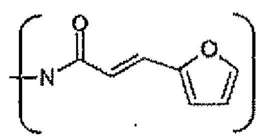
,

le groupement furane étant éventuellement remplacé par un autre radical hétérocycloalkyle choisi parmi un groupement thiophène, pyrrole, thiazole, pyrrazole ou oxazole, ledit groupement étant éventuellement substitué par un radical allyle ayant de 1 à 4 atomes de carbone, $NH_3$, O, OH, $CONH_2$, $OCH_3$, COOH, $COOCH_3$ ou $OCOCH_3$.

**7.** Composé selon la revendication 1, dans lequel dans la formule (b), $R_1$ est O, $R_2$ est $CH_3$, Y est H et -[$R_4$] est

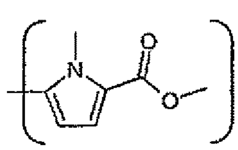
ou
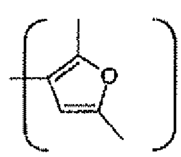
.

**8.** Composé selon la revendication 1, dans lequel dans la formule (a), $R_1$ est O ou NH, $R_2$ est $CH_3$, $R_3$ est NH, Y est H et R est NH-CO-$C_6H_5$.

**9.** Composé selon la revendication 1, dans lequel dans la formule (a), $R_1$ est O, $R_2$ est H ou $CH_3$, $R_3$ est S ou O, Y est H, R est NH-$(CH_2)_n$-$R_{12}$, n = 0 et -[$R_{12}$] est

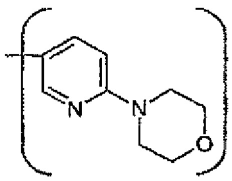,
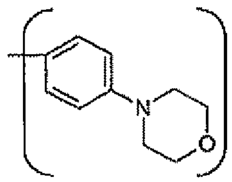,
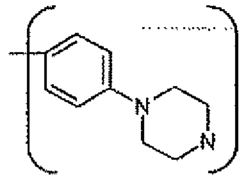,
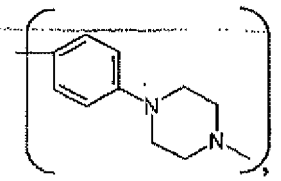,

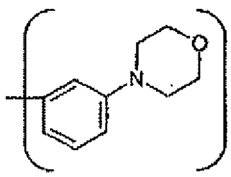,
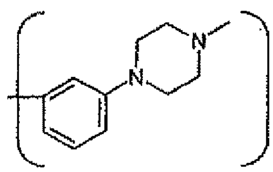,
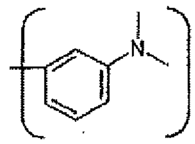
ou
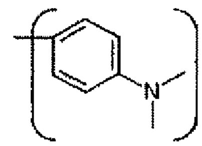.

**10.** Composé selon la revendication 1, dans lequel dans la formule (a), $R_1$ est O, $R_2$ est $CH_3$, $R_3$ est S, Y est H, R est NH-$(CH_2)_n$-$R_{12}$, n = 0 et $R_{12}$ est $C_6H_5$.

**11.** Composé selon la revendication 1, dans lequel dans la formule (a), $R_1$ est O, $R_2$ est $CH_3$, $R_3$ est S, Y est H et R est NH-CO-$R_8$ et $R_8$ est $CH_2$-$CH_2$-$C_4H_3O$ ou - [$R_8$] est

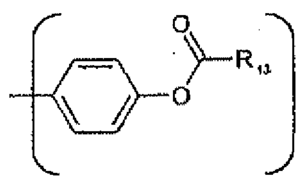,

$R_{13}$ étant un radical alkyle en $C_1$ à $C_6$.

**12.** Composé selon la revendication 1, dans lequel dans la annule (a), $R_1$ est O, $R_2$ est $CH_3$, $R_3$ est S, R est NH-$(CH_2)_n$-$R_7$, n = 1 et $R_7$ est $C_6H_5$.

**13.** Composé selon la revendication 1, dans lequel dans la formule (a'), $R_1$ est O, $R_2$ est $CH_3$, $R_3$ est $SCH_3$, et -[R] est

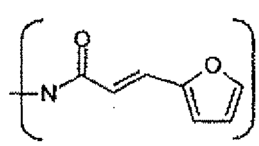

ou

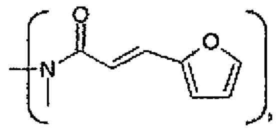

le groupement furane étant éventuellement remplacé par un autre radical hétérocycloalkyle choisi parmi un groupement thiophène, pyrrole, thiazole, pyrrazole ou oxazole, ledit groupement étant éventuellement substitué par un radical allyle ayant de 1 à 4 atomes de carbone, $NH_3$, O, OH, $CONH_2$, $OCH_3$, COOH, $COOCH_3$ ou $OCOCH_3$.

**14.** Composé selon la revendication 1, ayant l'une des formules suivantes :

Composé 03 : 1-[3-méthoxy-4-(2-oxo-2H-chromén-3-yl)phényl]-3-phénylurée,
Composé 04 : 1-[3-méthoxy-4-(2-oxo-2H-chromén-3-yl)phényl]-3-phénylthiourée,
Composé 05 : 4-fluoro-N-({[3-méthoxy-4-(2-oxo-2H-chromén-3-yl)phényl]-amino}-carbonothioyl)benzamide,
Composé 06: 1-[4-(diméthylamino)phényl]-3-[3-méthoxy-4-(2-oxo-2H-chromén-3-yl)-phényllurée,
Composé 07: N-(immo{[3-méthoxy-4-(2-oxo-2H-chromén-3-yl)phényl]amino}-méthyl)benzamide,
Composé 08: 1-[3-méthoxy-4-(2-oxo-2H-chromén-3-yl)phényl]-3-(6-morpholin-4-yl-pyridin-3-yl)thiourée,
Composé 09: 5-({[3-méthoxy-4-(2-oxo-2H-chromén-3-yl)phényl]amino}sulfonyl)-1-méthyl-1H-pyrrole-2-carboxylate de méthyle,
Composé 10 : N-[(2E)-3-(2-furyl)prop-2-énoyl]-N'-[3-méthoxy-4-(2-oxo-2H-chromén-3-yl)phényl]-N-méthylimidothiocarbamate de méthyle,
Composé 12: (2E)-3-(2-furyl)-N-(imino{[3-méthoxy-4-(2-oxo-2H-chromén-3-yl)-phényl]amino}méthyl)acrylamide,
Composé 13 : N-[3-méthoxy-4-(2-oxo-2H-chromén-3-yl)phényl]-2,5-diméthylfurane-3-sulfonamide,
Composé 14: N-[(2E)-3-(2-furyl)prop-2-énoyl]-N-[3-méthoxy-4-(2-oxo-2H-chromén-3-yl)phényl]imidothiocarbamate de méthyle,
Composé 15 : (2E)-3-(2-furyl)-N-({[3-méthoxy-4-(2-oxo-1,2-dihydroquinoléin-3-yl)-phényl]amino}carbonothioyl)acrylamide,
Composé 16 : N-(4-fluorobenzoyl)-N'-[3-méthoxy-4-(2-oxo-2H-chromén-3-yl)phényl]-N-méthylimidothiocarbamate de méthyle,
Composé 17: (2E)-3-(2-furyl)-N-({[3-méthoxy-4-(1-méthyl-2-oxo-1,2-dihydro-quinoléin-3-yl)phényl]amino}carbonothioyl)acrylamide,
Composé 18: 1-[3-(diméthylamino)phényl]-3-[3-méthoxy-4-(2-oxo-2H-chromén-3-yl)-phényl] urée,
Composé 19: N'-((E)-3-furan-2-yl-acryloyl)-N-[3-méthoxy-4-(2-oxo-2H-chromén-3-yl)phényl]-N-méthyl-guanidine,
Composé 20 : 1-[3-méthoxy-4-(2-oxo-2H-chromén-3-yl)-phényl]-3-(4-morpholin-4--yl-phényl)-thiourée,
Composé 21 : 1-((E)-3-furan-2-yl-acryloyl)-3-{4-[1-(2-hydroxy-éthyl)-2-oxo-1,2-dihydro- quinoléin-3-yl]-3-métlioxyphényl}thiourée,
Composé 22: N-benzoyl-N'-[3-méthoxy-4-(2-oxo-1,2-dihydroquinoléin-3-yl)-phényl]-guanidine,
Composé 23 : acétyl-[3-méthoxy-4-(2-oxo-2H-chromén-3-yl)-phényl]-amide de l'acide 2,5-diméthyl-furane-3-sulfonique,
Composé 24 : 1-((E)-3-furan-2-yl-acryloyl)-3-[3-méthoxy-4-(2-oxo-2H-chromén-3-yl)-phényl]-1,2-diméthyl-isothiourée, et
Composé 25 : 1-(4-fluorobenzoyl)-3-[3-méthoxy-4-(2-oxo-2H-chromén-3-yl)-phényl]-1,2-diméthylisothiourée,

**15.** Composé selon l'une quelconque des revendications 1 à 14, ayant des propriétés cytotoxiques sur les cellules tumorales.

**16.** Composé selon l'une quelconque des revendications 1 à 14, ayant des propriétés cytostatiques sur les cellules tumorales.

**17.** Composé selon la revendication 15 ou 16, dans lequel lesdites cellules tumorales sont des cellules tumorales de poumon, de prostate, de sein, de côlon, de mélanome, de lymphome ou myéloïdes.

**18.** Composé selon l'une quelconque des revendications 1 à 14, qui est soluble dans l'eau à une molarité supérieure à $50x10^{-6}$ mol/l.

**19.** Composé selon l'une quelconque des revendications 1 à 18, qui lie la protéine BCl-xL avec un Kd inférieur à 1 μM.

**20.** Composition comprenant un composé selon l'une quelconque des revendications 1 à 19 en association avec au moins un autre agent anti-tumoral.

**21.** Composition selon la revendication 20, dans laquelle ledit agent anti-tumoral est le taxol, l'étoposide, la doxorubicine, le cis-platine, l'acide rétinoïque, le tamoxifène, la vinblastine, la vincristine, le cyclophosphamide, le topotécan, l'irinotécan, la gemcitabine ou la rapamycine.

**22.** Produit contenant un composé selon l'une quelconque des revendications 1 à 19 et un autre agent anti-tumoral en tant que préparation combinée pour une utilisation simultanée, séparée ou séquentielle en thérapie.

**23.** Composition pharmaceutique comprenant un composé selon l'une quelconque des revendications 1 à 19 et un support pharmaceutiquement acceptable.

**24.** Composition pharmaceutique selon la revendication 23, qui est à administrer par injection intralésionnelle, intrapéritonéale, intramusculaire ou intraveineuse ; par perfusion; délivrance médiée par des liposomes ; délivrance topique, nasale, orale, annale, sous-cutanée, vaginale, sublinguale, urétrale, transdermique, intrathécale, oculaire ou otique.

**25.** Utilisation d'un composé selon l'une quelconque des revendications 1 à 19, pour la fabrication d'un médicament à administrer à un sujet pour traiter le cancer.

**26.** Utilisation selon la revendication 25, dans laquelle ledit médicament est à administrer par injection intralésionnelle, intrapéritonéale, intramusculaire ou intraveineuse ; par perfusion ; délivrance médiée par des liposomes ; délivrance topique, nasale, orale, anale, sous-cutanée, vaginale, sublinguale, urétrale, transdermique, intrathécale, oculaire ou otique.

**27.** Utilisation d'une composition selon la revendication 20 ou 21, pour la fabrication d'un médicament à administrer à un sujet pour traiter le cancer.

**28.** Utilisation d'un composé selon l'une quelconque des revendications 1 à 19, pour la fabrication d'un médicament à administrer simultanément, séparément ou séquentiellement avec au moins un autre agent anti-tumoral, à un sujet pour traiter le cancer.

**29.** Procédé d'inhibition de la prolifération de cellules *in vitro* comprenant la mise en contact d'une cellule *in vitro* avec un composé selon l'une quelconque des revendications 1 à 19.

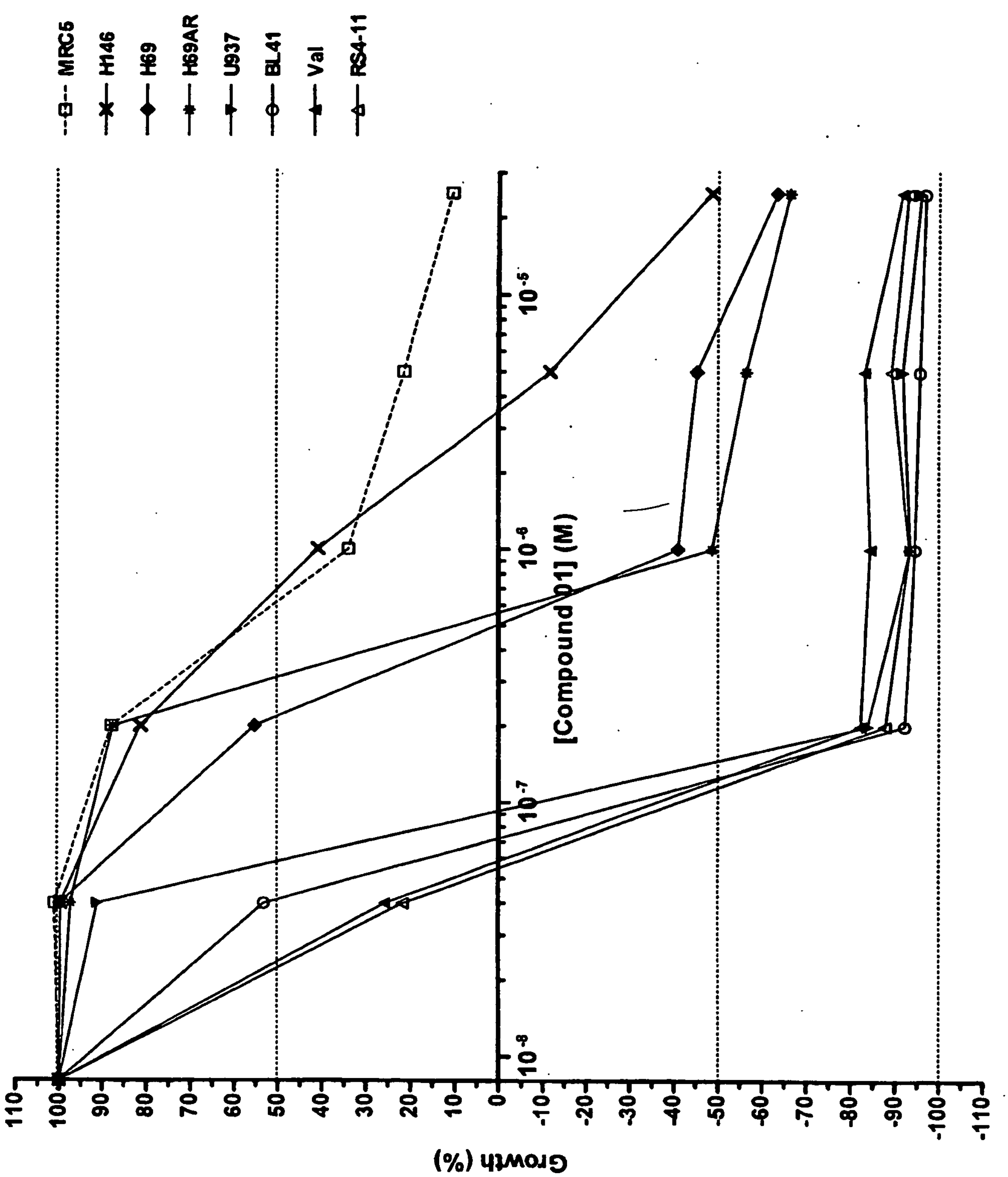


FIG. 1A

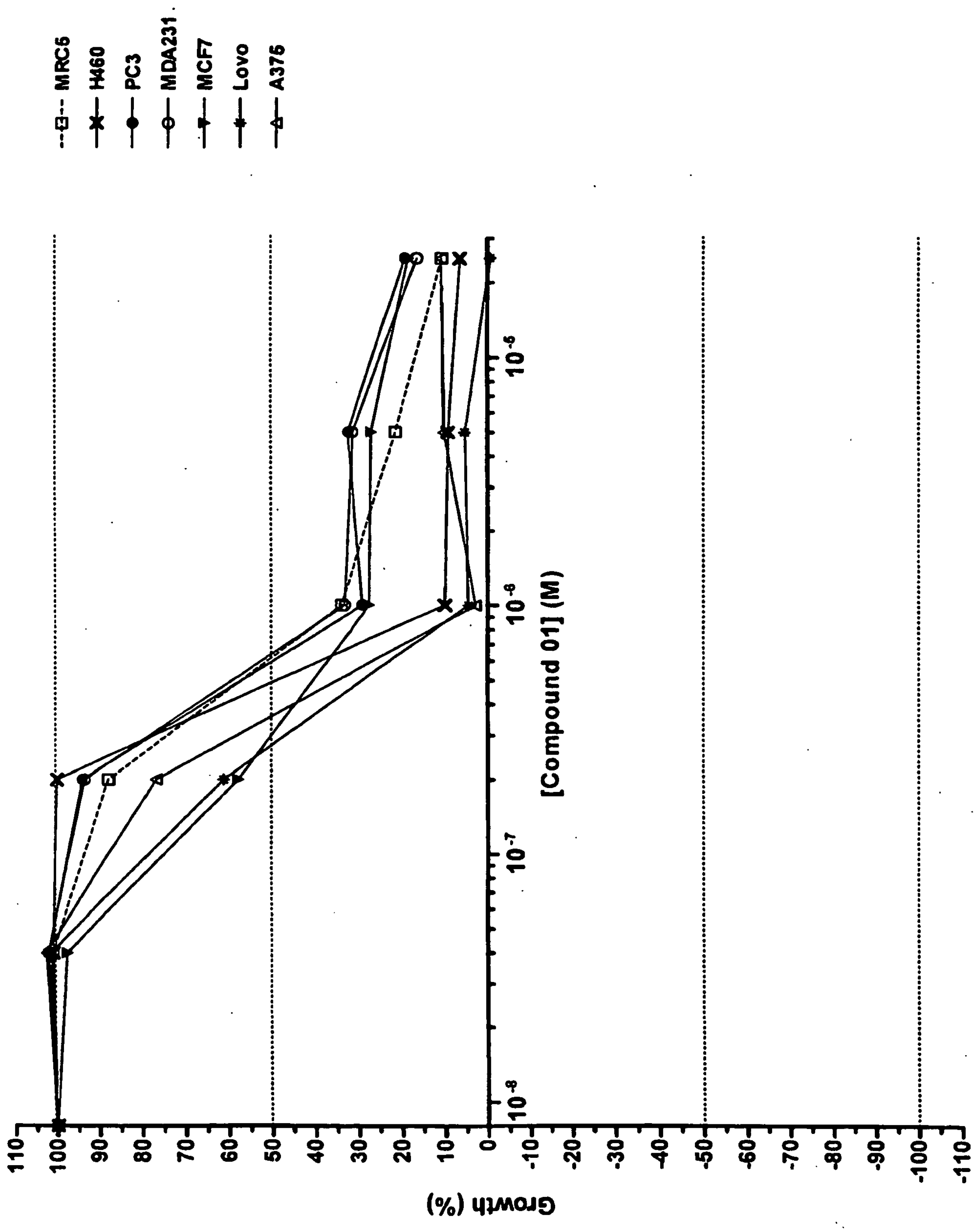


FIG. 1B

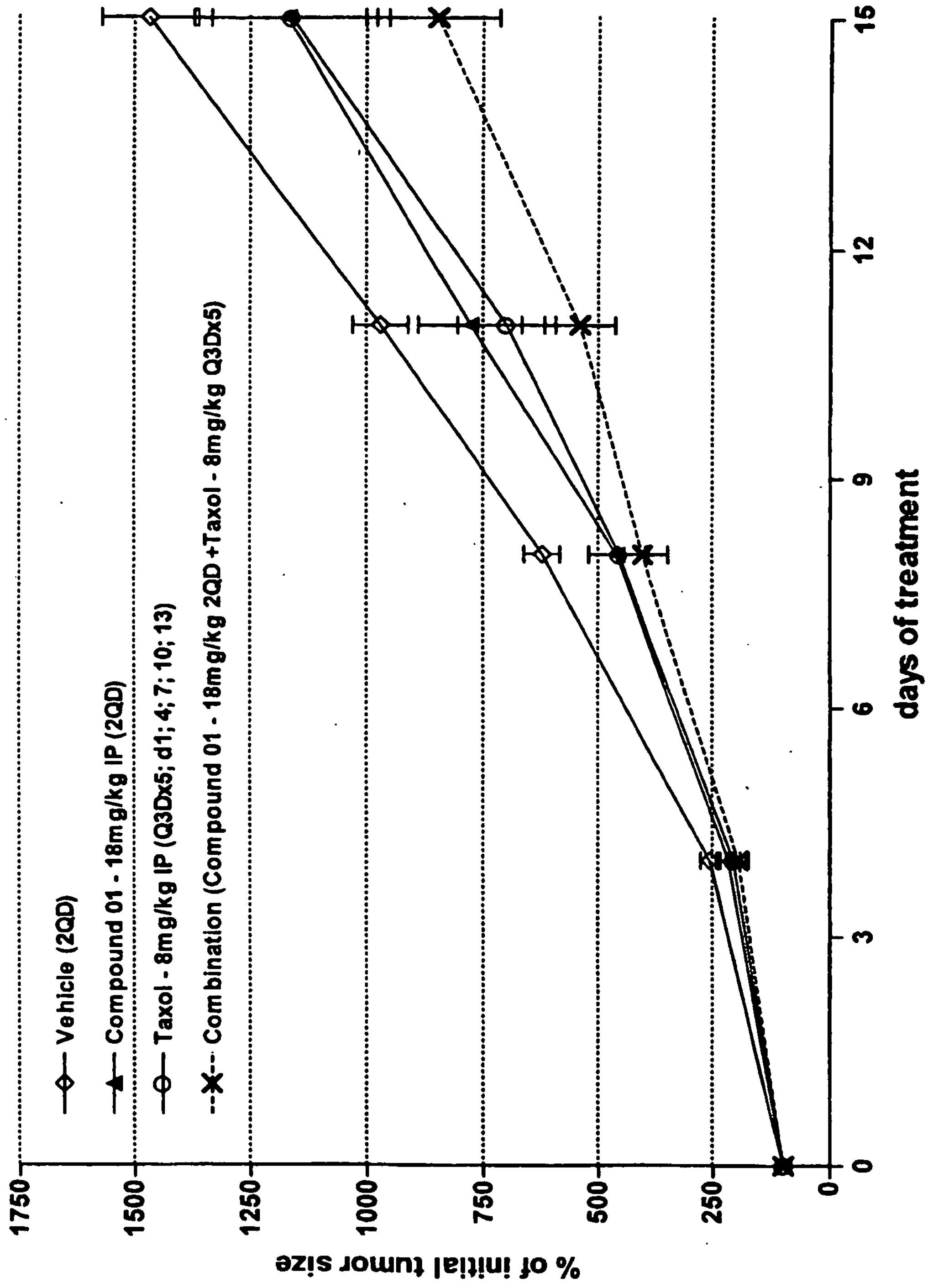
Vehicle (2QD)
Compound 01 - 18mg/kg IP (2QD)
Taxol - 8mg/kg IP (Q3Dx5; d1; 4; 7; 10; 13)
Combination (Compound 01 - 18mg/kg 2QD +Taxol - 8mg/kg Q3Dx5)
% of initial tumor size
1750
1500
1250
1000
750
500
250
0
0
3
6
9
12
15
days of treatment

FIG. 2

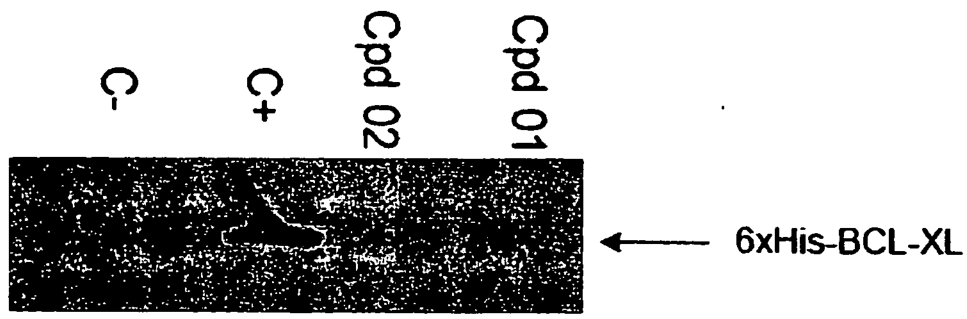


FIG. 3

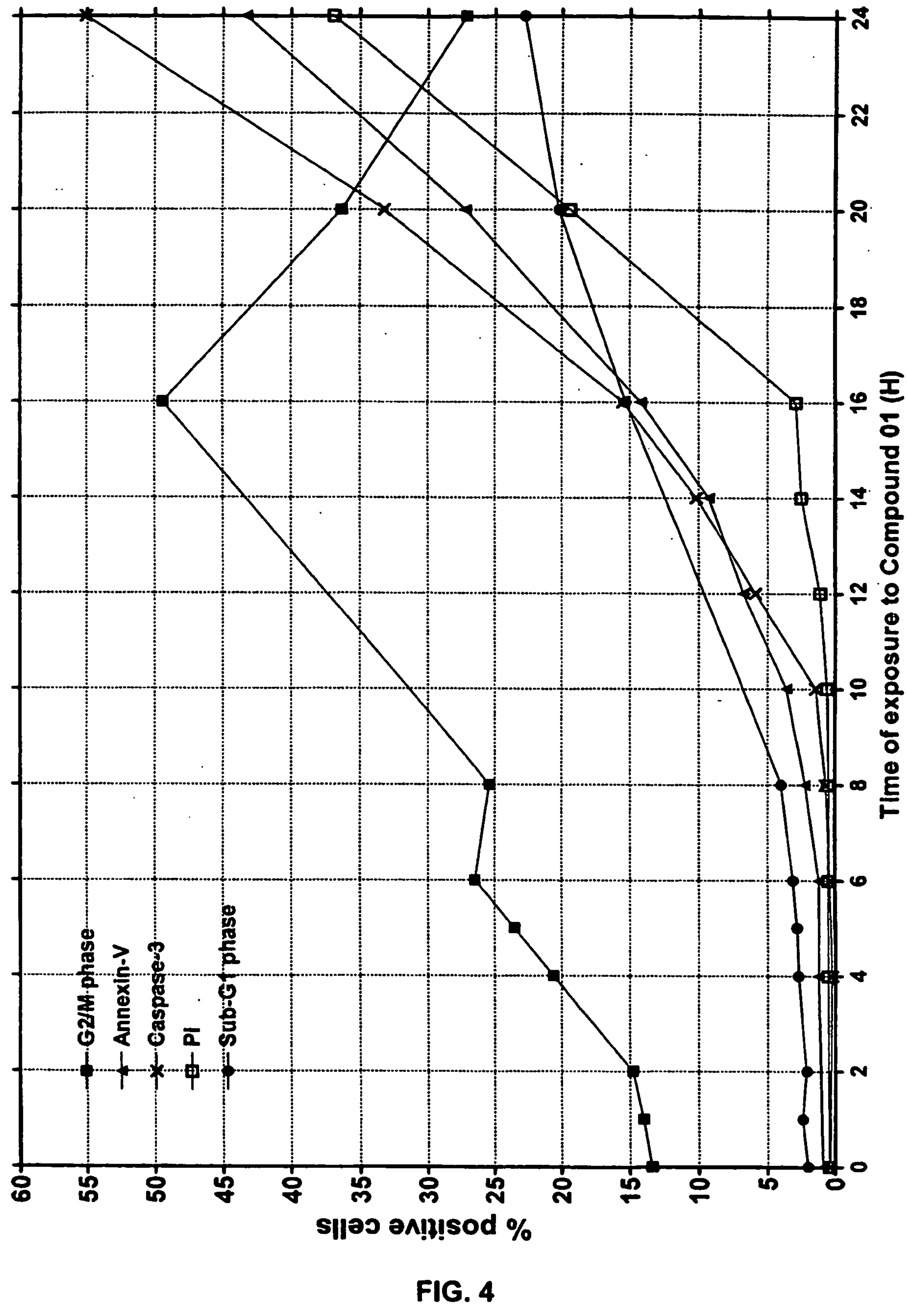
G2/M phase
Annexin-V
Caspase-3
PI
Sub-G1 phase
% positive cells
0 5 10 15 20 25 30 35 40 45 50 55 60
Time of exposure to Compound 01 (H)
0 2 4 6 8 10 12 14 16 18 20 22 24

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03020698 A2
- WO 0003990 A
- WO 2004072043 A

### Non-patent literature cited in the description


- *Org. Synth.,* vol. 3, 56
- **Contractor R ; Konopleva M ; Samudio I et al.** Inhibition of Bcl-2 signaling by small molecule BH3 inhibitor GX15-070 as a novel therapeutic strategy in AML. *Blood,* 2005, vol. 106, 942
- **Galan PP ; Roue G ; Billamor N et al.** The small molecule pan=Bcl-2 inhibitor GX15-070 induces apoptosis in vitro in mantle cell lymphoma (MCL) cells and exhibits a synergistic effect in combination with the proteasome inhibitor bortezomib (Velcade®). *Blood,* 2005, vol. 106, 429
- **Mohammad RM ; Wang S ; Aboukamee, A. ; Chen B ; Wu X ; Chen J ; Al-Katib A.** Preclinical studies of a nonpeptidic small-molecule inhibitor of Bcl-2 and Bcl-X(L) [(-)-gossypol] against diffuse large cell lymphoma. *Mol Cancer Ther,* January 2005, vol. 4 (1), 13-21
- **Oltersdorf T. et al.** An inhibitor of Bcl-2 family proteins induces regression of solid tumours. *Nature,* 2005, vol. 435 (7042), 677-81
- **Sattler M. et al.** Structure of Bcl-xL-Bak peptide complex: recognition between regulators of apoptosis. *Sicence,* 1997, vol. 275 (5302), 983-986